# EUROPEAN PATENT APPLICATION

(11) **EP 2 468 871 A1**
(43) Date of publication of application: **27.06.2012**
(21) Application number: 10016034.0
(22) Date of filing: 23.12.2010
(51) Int. Cl.: C12N 15/82, C07K 14/775

(54) **Method for producing apolipoprotein in plants**

(71) Applicant: Philip Morris Products S.A., 2000 Neuchâtel (CH)
(72) Inventor: The designation of the inventor has not yet been filed
(74) Representative: Lloyd, John Scott

(57) **Abstract**

The present invention provides, in one aspect, a method for producing Apolipoprotein in a plant comprising incubating or growing a plant into which has been introduced or infiltrated a nucleic acid construct comprising, consisting or consisting essentially of a nucleic acid sequence encoding an Apolipoprotein fusion protein that comprises a fusion protein partner that induces the formation of a protein body in a plant, preferably, wherein the step of introducing or infiltrating the plant is performed prior to the incubating or growing st ep.

## Description

### FIELD OF THE INVENTION

The present invention relates to a method for producing Apolipoprotein in a plant by accumulation thereof in protein bodies. Nucleic acid sequences, nucleic acid constructs, vectors, expression vectors and the like for carrying out the method are also disclosed.

### BACKGROUND OF THE INVENTION

In a healthy human, there is normally a balance between the delivery and removal of cholesterol. When people have a high level of low-density lipoprotein (LDL) and a low level of high-density lipoprotein (HDL), the imbalance results in more cholesterol being deposited in the arteries than that which is removed. Atherosclerosis is caused by the repeated deposit of cholesterol which leads to the narrowing or blocking of arteries. Apolipoprotein A1 (ApoA1) is the major protein constituent of HDL and plays an important role in cholesterol homeostasis and research suggests that ApoA1 and HDL can help to serve a protective role against this disease

In humans, ApoA1 is produced in the liver and intestinal cells as a non-glycosylated pre-pro-protein. The 18 amino acid pre-segment is removed before the protein leaves the cell and the 6 amino acid pro-segment is cleaved post secretion by an unknown protease in the plasma, leaving the mature 243 amino acid protein. Apolipoprotein A1 Milano (ApoA1-M) was the first described variant of human ApoA1 published in 1980. It is characterized by a substitution mutation of Arginine 173 with Cysteine. Individuals with this mutation have a significant reduction in their HDL-cholesterol levels and may be "protected" from atherosclerosis. The mechanism of this protective effect is though to be due to a modification in the structure of ApoA1-M, with the loss of one alpha-helix and an increased exposure of hydrophobic residues. This structure may cause an increased flexibility of the molecule, which associates more readily with lipids, compared to normal ApoA1.

The therapeutic use of Apolipoproteins and mutants thereof is limited by the lack of a method allowing the preparation of said Apolipoproteins in sufficient quantities economically. In particular, the recombinant production of Apolipoproteins is made difficult by its amphiphilic character, autoaggregation, and degradation.

The biochemical, technical, and economic limitations of existing prokaryotic and eukaryotic expression systems have created substantial interest in developing new and optimised production systems for heterologous proteins. To that end, plant expression systems can be used to produce recombinant proteins. However, a number of variables, including crop species selection, tissues choice, expression and recovery strategies and posttranslational processing have to be taken into consideration during the development and commercialization of a plant based production system. Accordingly, the development of a plant based production system is not straightforward and there is no certainty that the system that is eventually developed will be one that results in the effective production of the selected protein, especially on a commercial scale. In particular, problems are often encountered when purifying the recombinant protein from the plant expression system. This represents one of the most significant bottlenecks in recombinant protein production in plants. Protein purification from plants is a difficult task due to the complexity of the plant system. Plant solids are typically large, dense and relatively elevated at about 10-20% by weight. All of these problems are particularly acute in the context of the industrial production of recombinant proteins in plants, where multiple or complex steps may render the method unsuitable.

Current production systems for Apolipoprotein are not capable of producing Apolipoprotein at the levels required for clinical trials or therapeutic applications. Recombinant Apolipoprotein is also a challenging protein to express and produce, especially on a commercially useful scale. The method provided by the present invention meets this need.

### SUMMARY OF THE INVENTION

The present invention provides a method for producing Apolipoprotein in a plant based expression system, utilising a nucleic acid sequence that encodes a prolamin protein, preferably gamma-zein that induces the formation of a protein body in a plant. Advantageously, the expression of Apolipoprotein as a fusion protein with prolamin results in the increased expression of Apolipoprotein in a plant. The addition of one or more non-naturally occurring repeat sequence motifs to prolamin can increase the expression level of the Apolipoprotein as compared to the use of prolamin without the motif(s). The efficient expression of Apolipoprotein in plant protein bodies facilitates the recovery of the recombinant Apolipoprotein fusion protein. The methods described herein can be used to obtain Apolipoprotein in a substantially purified form on a commercial scale. The methods are therefore useful for producing substantially purified Apolipoprotein that is easily scalable for mass production of the protein.

In a first aspect, there is provided a method for producing Apolipoprotein in a plant comprising incubating or growing a plant into which has been introduced or infiltrated a nucleic acid construct comprising, consisting or consisting essentially of a nucleic acid sequence encoding an Apolipoprotein fusion protein that comprises a fusion protein partner that induces the formation of a protein body in a plant.

In a further aspect, there is provided a method for producing Apolipoprotein in a plant comprising the steps of: (a) incubating a plant into which has been introduced a nucleic acid construct comprising, consisting or consisting essentially of a nucleic acid sequence encoding a prolamin protein that induces the formation of a protein body in a plant, preferably gamma zein; and a nucleic acid sequence encoding Apolipoprotein, wherein said nucleic acid sequences are operably linked to each other; and (b) incubating said plant under conditions that allow for the expression of Apolipoprotein as a fusion protein in said plant.

In a further aspect, there is provided a method for expressing Apolipoprotein in a plant comprising the use of a nucleic acid construct comprising, consisting or consisting essentially of a nucleic acid sequence encoding a prolamin protein that induces the formation of a protein body in a plant, preferably, gamma zein; and a nucleic acid sequence encoding Apolipoprotein, wherein said nucleic acid sequences are operably linked to each other.

In a further aspect, there is provided a method for expressing Apolipoprotein in a plant comprising the steps of: (a) providing a plant comprising a nucleic acid construct comprising, consisting or consisting essentially of a nucleic acid sequence encoding a prolamin protein that induces the formation of a protein body in a plant, preferably gamma zein; and a nucleic acid sequence encoding Apolipoprotein, wherein said nucleic acid sequences are operably linked to each other; and (b) incubating said plant under conditions that allow for the expression of Apolipoprotein as a fusion protein in said plant.

In one embodiment, the step of introducing or infiltrating the plant is performed prior to the incubating or growing step.

In one embodiment, the nucleic acid construct used in the method comprises: a first nucleic acid sequence encoding a protein that induces the formation of a protein body in a plant optionally, further comprising a nucleic acid sequence encoding one or more non-naturally occurring repeat sequence motifs; optionally a second nucleic acid sequence encoding an amino acid linker in which a peptide bond therein can be specifically cleaved; and a third nucleic acid sequence encoding Apolipoprotein, and wherein said first, second and third nucleic acid sequences are operably linked to each other.

In one embodiment or combination of the above-mentioned embodiments of the method, the nucleic acid sequence further comprises a (fourth) nucleic acid sequence encoding a peptide that directs the fusion protein towards the endoplasmic reticulum of a plant cell, preferably a signal peptide.

In one embodiment or combination of the above-mentioned embodiments, the method comprises the additional step of: recovering the protein body comprising the fusion protein from the plant, preferably wherein said step comprises the steps of: (i) homogenising the plant material; (ii) centrifuging the homogenised plant material at low speed, preferably, about 200 x g; (iii) centrifuging the homogenised plant material at a higher speed than step (ii), preferably, about 6000 x g; and (iv) recovering the protein bodies comprising the fusion protein in the pelleted fraction.

In one embodiment or combination of the above-mentioned embodiments, the method comprises the further step of: solubilising the fusion protein, preferably, wherein said solubilisation step comprises the use of a mixture comprising, consisting or consisting essentially of a reducing agent , preferably, beta-mercaptoethanol, a non-ionic surfactant, preferably Triton X-100 and optionally a salt, preferably, sodium chloride.

In one embodiment or combination of the above-mentioned embodiments, the method comprises the further step of: releasing Apolipoprotein from said fusion protein, preferably, wherein a protease, preferably, TEV protease, or a protein splicing means, preferably an intein, is used to release Apolipoprotein from said fusion protein

In one embodiment or combination of the above-mentioned embodiments, the method comprises the further step of purifying the released Apolipoprotein.

In one embodiment or combination of the above-mentioned embodiments, said purifying step comprises:(i) contacting the fusion protein with an immobilized metal ion affinity chromatography column to immobilise the prolamin protein; (ii) eluting the Apolipoprotein; and (iii) further purifying the eluted Apolipoprotein using reversed phase chromatography, preferably reversed phase reversed phase fast protein liquid chromatography.

In a further aspect, there is provided a nucleic acid construct comprising, consisting or consisting essentially of: a first nucleic acid sequence encoding a protein that induces the formation of a protein body in a plant optionally, further comprising a nucleic acid sequence encoding one or more non-naturally occurring repeat sequence motifs; optionally a second nucleic acid sequence encoding an amino acid linker in which a peptide bond therein can be specifically cleaved; and a third nucleic acid sequence encoding Apolipoprotein, and wherein said first, second and third nucleic acid sequences are operably linked to each other.

In one embodiment or combination of the above-mentioned embodiments, the nucleic acid construct further comprises a regulatory nucleotide sequence that regulates the transcription of said nucleic acid sequence.

In a further aspect, there is provided a vector comprising the nucleic acid sequence or the nucleic acid construct.

In a further aspect, there is provided a fusion protein comprising, consisting or consisting essentially of: (i) an amino acid sequence encoding a prolamin protein that induces the formation of a protein body in a plant, preferably, gamma-zein; (ii) optionally an amino acid sequence encoding a cleavage recognition site; and (iii) an amino acid sequence encoding Apolipoprotein.

In a further aspect, there is provided a plant or plant material derived therefrom comprising the nucleic acid sequence, or the nucleic acid construct, or the vector, or the fusion protein. Suitably, the plant or plant material is a transformed or infiltrated plant or plant material.

In a further aspect, there is provided a plant protein body comprising the fusion protein.

In a further aspect, there is provided the use of the nucleic acid sequence, or the nucleic acid construct, or the vector for expressing and/or producing Apolipoprotein in a plant cell.

The embodiments and combinations of embodiments described above in relation to the method(s) for producing Apolipoprotein in a plant are also disclosed as embodiments of the further aspects described above.

Using the methods described herein, Apolipoprotein is expressed in plants at an increased level when fused to a nucleic acid sequence that encodes a prolamin protein, preferably, gamma-zein, to induce the formation of a protein body in a plant. The presence of one or more non-naturally occurring repeat sequence motifs in prolamin can further increase the expression level of Apolipoprotein.

The high level expression of recombinant Apolipoprotein in protein bodies protects the protein from proteolytic and enzymatic activities that may be present in the plant.

The protein bodies comprising recombinant Apolipoprotein fusion protein retain their high density which can simplify the recovery of Apolipoprotein protein.

The downstream recovery and purification protocol of the method may be simpler and less costly than current approaches for preparing recombinant Apolipoprotein.

The methods can be used for producing substantially purified Apolipoprotein on an industrial scale.

### DEFINITIONS

The technical terms and expressions used within the scope of this application are generally to be given the meaning commonly applied to them in the pertinent art of plant and molecular biology. All of the following term definitions apply to the complete content of this application. The word "comprising" does not exclude other elements or steps, and the indefinite article "a" or "an" does not exclude a plurality. A single step may fulfill the functions of several features recited in the claims. The terms "essentially", "about", "approximately" and the like in connection with an attribute or a value particularly also define exactly the attribute or exactly the value, respectively. The term "about" in the context of a given numerate value or range refers to a value or range that is within 20 %, within 10 %, or within 5 % of the given value or range.

"Homology, identity or similarity" refer to the degree of sequence similarity between two polypeptides or between two polynucleotide molecules compared by sequence alignment. The degree of similarity between two discrete polynucleotide sequences being compared is a function of the number of identical, or matching, nucleotides at comparable positions. The degree of similarity expressed in terms of percent identity may be determined by visual inspection and mathematical calculation. Alternatively, the percent identity of two polynucleotide sequences may be determined by comparing sequence information using the GAP computer program, version 6.0 described by Devereux et al. (Nucl. Acids Res. 12:387, 1984) and available from the University of Wisconsin Genetics Computer Group (UWGCG). Typical default parameters for the GAP program include: (1) a unary comparison matrix (comprising a value of 1 for identities and 0 for non-identities) for nucleotides, and the weighted comparison matrix of Gribskov and Burgess, Nucl. Acids Res. 14:6745, 1986, as described by Schwartz and Dayhoff, eds., Atlas of Protein Sequence and Structure, National Biomedical Research Foundation, pp. 353-358, 1979; (2) a penalty of 3.0 for each gap and an additional 0.10 penalty for each symbol in each gap; and (3) no penalty for end gaps. Various programs known to persons skilled in the art of sequence comparison can be alternatively utilized.

The term "upstream" refers to a relative direction/position with respect to a reference element along a linear polynucleotide sequence, which indicates a direction/ position towards the 5' end of the polynucleotide sequence. "Upstream" may be used interchangeably with the "5' end of a reference element."

The term "downstream" refers to a relative direction/position with respect to a reference element along a linear polynucleotide sequence, which indicates a direction/ position towards the 3' end of the polynucleotide sequence. "Downstream" may be used interchangeably with the "3' end of a reference element."

"Fragments" or "truncations" (for example, truncated proteins) include any portion of an amino acid sequence of a polypeptide which retains at least one structural or functional characteristic of the subject post-translational enzyme or polypeptide.

A "fusion protein" includes a protein in which a peptide sequence from a different protein is covalently linked together by one or more peptide bonds. A "fusion protein partner" refers to that portion of the fusion protein which induces the formation of a protein body in a plant.

The term "operably linked" refers to the joining of distinct DNA elements, fragments, or sequences to produce a functional transcriptional unit. Suitably, therefore, a regulatory sequence that regulates the transcription of said DNA elements, fragments, or sequences is positioned upstrea m thereof.

The terms "purify" and "isolate" and grammatical variations thereof, are used to mean the separation or removal, whether completely or partially, of at least one impurity from a mixture, which thereby improves the level of purity of Apolipoprotein in the composition.

"Transformation" refers to the alteration of genetic material of a cell resulting from the introduction of exogenous genetic material into the cell. A number of methods are available in the art for transforming a plant cell which are all encompassed herein, including biolistics, gene gun techniques, Agrobacterium-mediated transformation, viral vector-mediated transformation and electroporation. A transgenic plant can be made by regenerating plant cells that have been genetically transformed.

"Agroinfiltration" or "infiltration" is a method for inducing transient expression of genes in a plant or to produce a desired protein. In one aspect, the technique involves injecting a suspension of Agrobacterium cells into the underside of a plant leaf, where it transfers the desired gene to plant cells. Vacuum infiltration is another method for introducing large numbers of Agrobacterium cells into plant tissue. In this procedure, leaf disks, leaves, or whole plants are submerged in a container with the suspension, and the container is placed in a vacuum chamber. The vacuum is then applied which causes air to exit through the stomata. When the vacuum is released, the pressure difference forces the suspension through the stomata and into the plant tissue.

The term "plant" refers to any plant at any stage of its life cycle or development, and its progenies. The plant may be or may be derived from a naturally occurring, mutant, non-naturally occurring or transgenic plant.

The term "plant cell" refers to a structural and physiological unit of a plant. The plant cell may be in form of a protoplast without a cell wall, an isolated single cell, a cultured cell, a clump of two or more cells or as a part of higher organized unit such as but not limited to, plant tissue, a plant organ, or a whole plant.

The term "plant material" refers to any solid, or liquid composition, or a combination thereof, obtained or obtainable from a plant, including leaves, stems, roots, flowers or flower parts, fruits, pollen, egg cells, zygotes, seeds, cuttings, secretions, extracts, cell or tissue cultures, or any other parts or products of a plant. In one embodiment, the plant material is or is derived from a leaf - such as a green leaf.

### DETAILED DESCRIPTION

The method for producing Apolipoprotein in a plant comprises a first step of incubating a plant into which has been introduced a nucleic acid construct comprising a nucleic acid sequence that encodes a protein that induces the formation of a protein body in a plant, wherein said protein that induces the formation of a protein body in a plant is prolamin, preferably, gamma-zein; and a nucleic acid sequence encoding Apolipoprotein, wherein said nucleic acid sequences are operably linked to each other.

The nucleic acid sequence encoding Apolipoprotein encompass nucleic acid sequences with substantial homology (that is, sequence similarity) or substantial identity to the nucleic acid sequence of Apolipoprotein, including any mammalian (for example, human) Apolipoprotein and any nucleic acid sequences that encode pro- Apolipoprotein and pre-pro- Apolipoprotein. As used herein, the term "pro-Apolipoprotein" refers to an apolipoprotein polypeptide which includes a polypeptide which is cleaved post-translationally. The term "pre-pro- Apolipoprotein" refers to a pro- Apolipoprotein molecule additionally comprising an N-terminal signal sequence which facilitates intracellular transport of the polypeptide chain. In one embodiment, the nucleic acid sequence encodes pro-apolipoprotein.

Classes of Apolipoprotein are also encompassed in the present invention, including Apolipoprotein A, Apolipoprotein AI, Apolipoprotein AII Apolipoprotein A-IV, Apolipoprotein A-V, Apolipoprotein B, Apolipoprotein B-100, Apolipoprotein B-48, Apolipoprotein C-I, Apolipoprotein C-II, Apolipoprotein C-III, Apolipoprotein C-IV, Apolipoprotein D, Apolipoprotein E Apolipoprotein F, Apolipoprotein H and Apolipoprotein L. Exemplary nucleic acid sequences encoding Apolipoprotein are well known to the art and are generally readily available from a diverse variety of mammalian sources including human, porcine, bovine, ovine and the like. In a preferred embodiment, the Apolipoprotein is Apolipoprotein A1, preferably, human Apolipoprotein A1. The nucleic acid and amino acid sequences of these Apolipoproteins are widely available in databases - such as Genbank.

In one embodiment, the nucleic acid sequence of the mature peptide of Apolipoprotein A1-Milano is a coding sequence which has been optimised for expression in plants and comprises, consists or consists essentially the sequence set forth in SEQ ID No. 1 or is a variant, fragment or homologue thereof. In one embodiment, the amino acid sequence of the mature peptide of Apolipoprotein A1-Milano comprises, consists or consists essentially the sequence set forth in SEQ ID No. 2 or is a variant, fragment or homologue thereof.

Apolipoprotein variants are also encompassed in the present invention and include human Apolipoprotein wherein a variety of natural and/or synthetic mutations and modifications have been discovered including, but not limited to, point mutations, deletion mutations, frameshift mutations and chemical modifications. In accordance with the present invention in a preferred embodiment, the Apolipoprotein variant is a mutant Apolipoprotein produced in a plant, preferably, Apolipoprotein Al-Milano. Other variants of Apolipoproteins comprising genetic polymorphisms are also known and are encompassed within the scope of the present invention. The expressed Apolipoprotein may be in the form of a monomer, a dimer or a trimer.

The variant of Apolipoprotein may have deletions, insertions or substitutions of amino acid residues, which produce a silent change and result in a functionally equivalent protein. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine. Conservative substitutions may be made, for example according to the Table below. Amino acids in the same block in the second column and preferably in the same line in the third column may be substituted for each other:

| | | |
|---|---|---|
| ALIPHATIC | Non-polar | Gly Ala Pro Ile Leu Val |
| | Polar - uncharged | Cys Ser Thr Met Asn Gly |
| | Polar - charged | Asp Glu Lys Arg |
| AROMATIC | | His Phe TrpTyr |

Alterations to the nucleic acid sequence encoding Apolipoprotein to prepare apolipoprotein variants may be made using a variety of nucleic acid modification techniques known to those skilled in the art, including for example site directed mutagenesis, targeted mutagenesis, random mutagenesis, the addition of organic solvents, gene shuffling or a combination of these and other techniques known to those of skill in the art.

A variant of Apolipoprotein may have at least about 60%, 65%, 70%, 71%, 72%, 73%, 74%, 75%, 80%, 85%, 90%, 95%, 96%, 97%, 98%, 99% or 100% sequence identity to the native or wild-type sequence of the Apolipoprotein from which the variant is derived. The Apolipoprotein variant may be a variant of Apolipoprotein displaying the biological and/or immunological activity of the native or wild-type Apolipoprotein. As used herein, the phrase "biological activity of an Apolipoprotein " means that the Apolipoprotein-like polypeptide has at least one biological activity which is substantially the same as or is similar to the naturally occurring or wild type Apolipoprotein. As used herein, the phrase "immunological activity of an Apolipoprotein " refers to the ability of an Apolipoprotein variant to cross-react with an antibody which is specific for a naturally occurring or wild type Apolipoprotein. An example of such an antibody is disclosed in US 6,828,114 which describes a monoclonal antibody that reacts specifically with ApoA1 and US 6,096,516 describes murine antibodies against apolipoprotein B-100.

The activity of Apolipoprotein can be conveniently measured using methods known in the art. By way of example, the ability to improve the reverse cholesterol transport in a mammal can be measured. Measurement of reverse cholesterol transport capacity can be determined by calculation of the association kinetics of the Apolipoprotein with dimyristoylphosphatidylcholine (DMPC) (see Biochem. Biophys. Acta (1997) 1344:139). Briefly, the DMPC is resuspended in 100 mM NaCl, 50 mM Tris-HCl pH 8.0 and 0.25 mM EDTA at a concentration of 0.5 mg/ml. The apolipoprotein, a buffer containing 2 mM 5' -5' dithiobisnitrobenzoate (NbS), and the DMPC suspension are incubated at 24 °C for 10 min then mixed to have a final DMPC concentration of 0.4 mg/ml and a protein concentration of 5.2 mM. The change in turbidity of the mix (absorbance) reflects a change in the lipid-protein association and is followed by measuring the absorbance at 325 nm. As control the same test can be repeated using a reduction buffer containing 20 mM 2-mercaptoethanol or 10 mM dithithreitol (DTT).

The invention also encompasses the use of a nucleic acid sequence that encodes Apolipoprotein or a fragment thereof. The nucleic acid sequence may not be identical to the naturally occurring Apolipoprotein so long as it encodes Apolipoprotein or a variant of Apolipoprotein. Accordingly, the nucleic acid sequence may have at least 80%, 85%, 90%, 95%, 96%, 97%, 98%, 99% or 100% sequence identity relative to the naturally occurring coding sequence of Apolipoprotein. In a preferred embodiment, the nucleic acid sequence encoding Apolipoprotein used in the invention has been optimized for expression in plants by substituting certain codons with alternative codons in accordance with the codon usage in plants.

A nucleic acid sequence encoding a protein that induces the formation of a protein body in a plant is also used in the methods of the invention. Protein bodies are naturally-occurring structures in certain plant seeds that have evolved to concentrate storage proteins intracellularly in eukaryotic cells while retaining correct folding and biological activity. Protein bodies share some of the characteristics of the inclusion bodies from bacteria. They are dense, and contain a high quantity of aggregated proteins that are tightly packed by hydrophobic interactions. The storage proteins can be inserted into the lumen of the endoplasmic reticulum via a signal peptide and are assembled either in the endoplasmic reticulum developing specific organelles called endoplasmic reticulum-derived protein bodies or in protein storage vacuoles. The protein that induces the formation of a protein body in a plant is a prolamin, including a modified prolamin or one or more prolamin domains. Suitably, the prolamin is a maize prolamin. Alpha and gamma-zeins are two non-limiting examples of maize prolamins. Alpha- and gamma-zeins are biosynthesized in membrane-bound polysomes at the cytoplasmic side of the rough endoplasmic reticulum, assembled within the lumen and then sequestered into endoplasmic reticulum-derived protein bodies.

Suitable prolamins include, but are not limited to, alpha-zein (for example, the 22 kDa N-terminal fragment of the maize alpha-zein), gamma-zein, alpha gliadin and the rice rP13 protein.

In a preferred embodiment, the protein that induces the formation of a protein body in a plant is gamma zein, preferably, maize gamma-zein, which is composed of four characteristic domains i) a peptide signal of 19 amino acids, ii) the repeat domain comprising eight units of the hexapeptide PPPVHL (53 aa), iii) the ProX domain where proline residues alternate with other amino acids (29 aa) and iv) the hydrophobic cysteine rich C-terminal domain.

One or more non-naturally occurring repeat sequence motifs can be incorporated or substituted into gamma-zein which may improve the expression level of Apolipoprotein in a plant cell. Where the non-naturally occurring repeat sequence motif(s) are substituted, the repeat domain or the ProX domain or both, of these domains are mutated to create the non-naturally occurring sequence motif. Since the repeat sequence is a non-naturally occurring sequence motif then it will not be present in the native gamma-zein (for example, native maize gamma zein) sequence. In one embodiment, the non-naturally occurring repeat sequence motif(s) are incorporated or substituted into the repeat domain of gamma-zein. In another embodiment, the non-naturally occurring repeat sequence motif(s) are incorporated into the ProX domain of gamma-zein. In another embodiment, the non-naturally occurring repeat sequence motif(s) are incorporated into the repeat domain and the ProX domain of gamma-zein In a preferred embodiment, the non-naturally occurring repeat sequence motif(s) are substituted into a fragment which consists essentially of the repeat domain and the ProX domain of gamma-zein. An example of such a fragment comprises, consists or consists essentially of at least amino acid residues 22 to 109, 22 to 110, 22 to 111, 22 to 112, 22 to 113, 22 to 114 or 22 to 115 of gamma-zein. In other words, the N-terminus of the fusion protein partner comprises, including the signal peptide of gamma-zein, the first 105 to 115 amino acids of gamma-zein with various substitutions as described in the foregoing.

Non-limiting examples of the non-naturally occurring repeat sequence motifs are selected from the group consisting of: (PPPVAL)n or (Pro Pro Pro Val Ala Leu)n; (PPPVEL)n or (Pro Pro Pro Val Glu Leu)n; (PPPAPA)n or (Pro Pro Pro Ala Pro Ala)n; and (PPPEPE)n or (Pro Pro Pro Glu Pro Glu)n or a combination of two or more thereof, wherein n = 1 to 5, 1 to 6, 1 to 7, 1 to 8, 1 to 9, 1 to 10, 1 to 15, 1 to 20 or 1 to 25 and so on. In a preferred embodiment, n = 7 or 8. Beside, alanine and glutamate, other amino acids (such as but not limited to threonine) can also be used in the proline-rich non-naturally repeat sequence, (for example, (PPPVTL)).

In another embodiment, combinations of two or more of different non-naturally occurring repeat sequence motifs can be used in the repeat domain, the ProX domain or both - such as (PPPVAL)n and (PPPVEL)n; or (PPPAPA)n and (PPPEPE)n; or (PPPVAL)n and (PPPVEL)n and (PPPAPA)n; or (PPPVEL)n and (PPPAPA)n and (PPPEPE)n; or (PPPVAL)n and (PPPVEL)n and (PPPAPA)n and (PPPEPE)n.

In one embodiment, the (PPPAPA)n sequence in the repeat domain of gamma zein comprises, consists or consists essentially of the sequence set forth in SEQ ID No. 6.

In one embodiment, the (PPPEPE)n sequence in the repeat domain of gamma zein comprises, consists or consists essentially of the sequence set forth in SEQ ID No. 7.

In one embodiment, the (PPPVEL)n sequence in the repeat domain of gamma zein comprises, consists or consists essentially of the sequence set forth in SEQ ID No. 8.

In one embodiment, the (PPPVAL)n sequence in the repeat domain of gamma zein comprises, consists or consists essentially of the sequence set forth in SEQ ID No. 9.

In one embodiment, the (PPPVTL)n sequence in the repeat domain of gamma zein comprises, consists or consists essentially of the sequence set forth in SEQ ID No. 10.

In one embodiment, the (PPPAPA)n sequence in the ProX domain of gamma-zein comprises, consists or consists essentially of the sequence set forth in SEQ ID No. 11.

In one embodiment, the (PPPEPE)n sequence in the ProX domain of gamma-zein comprises, consists or consists essentially of the sequence set forth in SEQ ID No. 12.

The non-naturally occurring repeat sequence motif(s) may be positioned at the 5' or the 3'-end of the repeat domain and/or the ProX domain of gamma-zein. The non-naturally occurring repeat sequence motif(s) may be positioned at the 5' and the 3'-end of the repeat domain and/or the ProX domain of gamma-zein. In a suitable embodiment, the non-naturally occurring repeat sequence motif(s) is positioned within the repeat domain and/or the ProX domain of gamma-zein. Suitably, said plant cell does not produce protein bodies in the absence of the nucleic acid encoding the fusion protein.

In one embodiment, maize gamma zein comprises the nucleic sequence set forth in SEQ ID No. 3 or the amino acid sequence set forth in SEQ ID No. 4. In another embodiment, the amino acid sequence of a fragment of gamma zein comprises the sequence set forth in SEQ ID No. 5 or is a variant, fragment or homologue thereof. Mutants, variants, fragments and homologues of these sequence are encompassed within the present invention.

Suitably, said plant cell does not produce protein bodies in the absence of the nucleic acid encoding the fusion protein.

Suitably, the protein body-inducing sequence further includes a sequence that directs a protein towards the endoplasmic reticulum of a plant cell. The sequence is often referred to as a leader sequence or signal peptide and can be from the same plant in which the fusion protein is expressed or a different plant. Examples of signal peptides are the 19 residue gamma-zein signal peptide sequence (see WO 2004003207); the 19 residue signal peptide sequence of alpha-gliadin or the 21 residue gamma-gliadin signal peptide sequence (see, for example, Plant Cell (1993) 5:443-450 and EMBO J (1984) 3 (6), 1409-11415). Similarly functioning signal peptides from other plants are also reported in the literature. The signal peptide may be a signal peptide that is native to gamma zein and/or Apolipoprotein. The nucleic acid encoding the signal peptide may be positioned in a nucleic acid construct such that it is expressed at the N- or the C-terminus of the protein. In one embodiment, the signal peptide is expressed at the N-terminus.

Thus, according to one embodiment, a nucleic acid molecule comprising the nucleic acid sequence that encodes a protein that induces the formation of a protein body in a plant (for example, prolamin, maize prolamin, gamma-zein or maize gamma-zein); a nucleic acid sequence encoding Apolipoprotein (for example ApoA1 or ApoA1 Milano); optionally a nucleic acid sequence encoding the gamma-zein signal peptide sequence; and optionally a nucleic acid sequence encoding a spacer is described herein. Suitably, said nucleic acid sequences are operably linked to each other.

Variants with substantial homology (that is, sequence similarity) or substantial identity to gamma-zein are also encompassed herein. A variant of a gamma-zein polynucleotide may have at least 60%, 65%, 70%, 71%, 72%, 73%, 74%, 75%, 80%, 85%, 90%, 95%, 96%, 97%, 98%, 99% or 100% sequence identity to the sequence reported in Genbank accession number NM_001111884. This term also encompasses fragments of gamma-zein with substantial homology (that is, sequence similarity) or substantial identity thereto. The gamma-zein variant may be a variant displaying the biological and/or immunological activity of gamma-zein. As used herein, the phrase "biological activity of gamma-zein" means that the gamma-zein variant has at least one biological activity which is substantially the same as or is similar to naturally occurring gamma-zein. As used herein, the phrase "immunological activity of gamma-zein " refers to the ability of a gamma-zein variant to cross-react with an antibody which is specific for a naturally occurring gamma-zein. Variants of gamma-zein may include amino acids in addition to those of a native gamma-zein protein or it may not include all of the amino acids of native gamma-zein protein. The gamma-zein may be a fragment of gamma-zein protein, said fragment comprising a nucleotide sequence that encodes a protein that induces the formation of a protein body in a plant. Thus, by way of example, gamma-zein may encode all or part of the repetition domain of the protein gamma-zein or all or part of the ProX domain.

The variant of gamma-zein may have deletions, insertions or substitutions of amino acid residues, which produce a silent change and result in a functionally equivalent protein. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine. Conservative substitutions may be made, for example according to the Table below. Amino acids in the same block in the second column and preferably in the same line in the third column may be substituted for each other:

| | | |
|---|---|---|
| ALIPHATIC | Non-polar | Gly Ala Pro Ile Leu Val |
| | Polar - uncharged | Cys Ser Thr Met Asn Gly |
| | Polar - charged | Asp Glu Lys Arg |
| AROMATIC | | His Phe TrpTyr |

Suitably, the nucleic acid sequences are operably linked to each other such that a fusion protein comprising Apolipoprotein and gamma-zein is expressed in a plant cell. In one embodiment, the nucleic acid sequence comprises Apolipoprotein at the 5' end and gamma-zein at the 3' end. In another embodiment, the nucleic acid sequence comprises Apolipoprotein at the 3' end and gamma-zein at the 5' end.

Suitably, the nucleic acid molecule includes a linker sequence between the nucleic acid sequence that induces the formation of a protein body in a plant and the nucleic acid sequence encoding Apolipoprotein. Said linker sequence may be operably linked thereto. In one embodiment, the linker sequence encodes an amino acid linker in which one or more peptide bonds therein can be specifically cleaved. It may therefore function as a recognition site for an enzyme or an intein and the like such that the two proteins can be separated from each other. The linker can be cleaved by any entity which can specifically cleave one or more peptide bonds. Advantageously, the linker allows for the separation of Apolipoprotein from the fusion protein which allows Apolipoprotein to be subsequently purified if desired to thereby provide a substantially homogeneous recombinant Apolipoprotein protein. Suitably, Apolipoprotein is not internally cleaved and so fragments of Apolipoprotein are not created. Suitably, Apolipoprotein is cleaved without leaving residual amino acids at the N-terminus or the C-terminus of Apolipoprotein. In one embodiment, the fusion protein is not cleaved with enterokinase since this cleaves Apolipoprotein internally.

A preferred method of cleaving the fusion protein to release Apolipoprotein is to design the fusion protein in such a way that the N-terminus of the fusion partner is linked to the C-terminus of Apolipoprotein via an amino acid linker in which a peptide bond therein can be specifically cleaved and wherein the amino acid linker does not occur elsewhere in the fusion protein. This approach has the advantage that the cleavage means can by chosen by reference to a specific amino acid sequence - such as a specific recognition sequence. The linker may contain more than the absolute minimum sequence necessary to direct specific cleavage of one or more peptides bonds. The linker may be generated as a result of the union between two nucleic acid sequences. In this embodiment, each sequence contains a number of nucleotides which can become ligated to form a cleavable linker - such as a cleavable recognition site.

In one embodiment, the fusion protein is not cleaved with enterokinase since this cleaves Apolipoprotein internally. However, this cleavage site may still be used to improve the expression of Apolipoprotein, especially when a protease is not used for cleavage and is replaced with a chemical or an intein for example. In another embodiment, the fusion protein is not cleaved with a Glu-C endoproteases, also known as *Staphylococcus aureus* Protease V8. This protease is a serine protease which selectively cleaves peptide bonds C-terminal to glutamic acid residues. The protease also cleaves at aspartic acid residues at a rate 100-300 times slower than at glutamic acid residues. However, the presence of a cleavage recognition site for this protease can decrease the level of expression of Apolipoprotein as compared to the use of gamma-zein alone.

A preferred method of cleaving the fusion protein to release Apolipoprotein is to design the fusion protein in such a way that the N-terminus of the fusion partner is linked to the C-terminus of Apolipoprotein via a sequence of amino acids which include a specific recognition site for enzymatic cleavage which does not occur elsewhere in the fusion protein. This approach has the advantage that the cleavage enzyme can by chosen by reference to its recognition sequence. The recognition site may contain more than the absolute minimum sequence necessary to direct cleavage. The recognition site may be generated as a result of the union between two nucleic acid sequences. In this embodiment, each sequence contains a number of nucleotides which can become linked to form a cleavable recognition site.

The nucleic acid sequences described herein may be plant optimised sequences.

A protease may be used to specifically cleave one or more peptide bonds in the linker. The protease may be Ala-C endoprotease. In another embodiment, the protease is Glu-C endoprotease, also known as *Staphylococcus aureus* Protease V8. This protease is a serine protease which selectively cleaves peptide bonds C-terminal to glutamic acid residues. The protease also cleaves at aspartic acid residues at a rate 100-300 times slower than at glutamic acid residues.

According to a further embodiment, a linker sequence may be added between the nucleic acid sequence that encodes a protein that induces the formation of a protein body in a plant and the cleavage recognition site. According to another embodiment, a linker sequence may be added between the cleavage recognition site and Apolipoprotein. The linker may function to improve cleavage efficiency. Non-limiting examples of linker sequences include the amino acid linkers (Gly)5 or (Gly4Ser)3.

In another embodiment, the protease is TEV protease. TEV protease is a highly site-specific cysteine protease that is found in the Tobacco Etch Virus. The optimum cleavage recognition site for this protease is the sequence Glu-Asn-Leu-Tyr-Phe-Gln-(Gly/Ser) and cleavage occurs between the Gln and Gly/Ser residues.

Non-limiting examples of suitable linkers therefore include Glu-Asn-Leu-Tyr-Phe-Gln-(Gly/Ser), (Gly)x, wherein x is 2, 3, 4, 5, 6, 7, 8, 9 or 10 or more or (Gly4Ser)y, wherein y is 2, 3, 4, 5, 6, 7, 8, 9 or 10 or more. In one embodiment, the linker is (Gly)4. In another embodiment the linker is (Gly4Ser)3. In a further embodiment, the sequence encoding A polipoprotein is located at the 3'-end of said linker.

In one preferred embodiment, the protease that is used to cleave the cleavage recognition site is TEV protease. TEV protease is a highly site-specific cysteine protease that is found in the Tobacco Etch Virus. The optimum cleavage recognition site for this protease is the sequence Glu-Asn-Leu-Tyr-Phe-Gln-(Gly/Ser) (ENLYFQ(G/S)) and cleavage occurs between the Gln and Gly/Ser residues. In one embodiment, the cleavage recognition site for Glu-C endoproteases or pepstatin-sensitive aspartyl (PAPA) protease is used together with TEV protease. In one embodiment, a linker between protein that induces the formation of a protein body in a plant and the cleavage recognition site may also be present - such as (Gly)5 or (Gly4Ser)3.

According to another embodiment, non-proteolytic means may be used to separate the two proteins. Thus, for example, inteins may be used. A variety of different inteins are known in the art, in which cleavage can be induced under defined conditions - such as reducing conditions. Thus, according to one embodiment, the amino acid linker may encode an intein. The intein may be derived from various bacterial species - such as Synechocystis sp. or Mycobacterium sp. - such as *Mycobacterium xenopi*, for example. The intein may be derived from Saccharomyces sp. - such as *Saccharomyces cerevisiae*, for example, the *Saccharomyces cerevisiae* vacuolar membrane ATPase intein. In one embodiment, the intein is a *Mycobacterium xenopi* Gyrase A intein. Chemicals may also be used to separate Apolipoprotein from the fusion protein in which case an amino acid linker may not be required.

The nucleic acid sequences described herein may be plant optimised sequences.

According to a further embodiment, the nucleic acid construct for use in the method of the present invention comprises: a first nucleic acid sequence encoding a protein that induces the formation of a protein body in a plant optionally, further comprising a nucleic acid sequence encoding one or more non-naturally occurring repeat sequence motifs; optionally a second nucleic acid sequence encoding an amino acid linker in which a peptide bond therein can be specifically cleaved; and a third nucleic acid sequence encoding Apolipoprotein, and wherein said first, second and third nucleic acid sequences are operably linked to each other. Optionally, a regulatory nucleotide sequence that regulates the transcription of said nucleic acid sequences is also included.

Various regulatory nucleotide sequences that regulates the transcription of said nucleic acid sequences may therefore also be included. These include transcriptional control elements that are only active in particular cells or tissues at specific times during plant development, such as in vegetative tissues or reproductive tissues. One such example is a promoter which refers to a polynucleotide element/sequence, typically positioned upstream and operably-linked to a double-stranded DNA fragment. A suitable promoter will enable the transcriptional activation of a nucleic acid sequence by recruiting the transcriptional complex, including the RNA polymerase and various factors, to initiate RNA synthesis. Promoters can be derived entirely from regions proximate to a native gene, or can be composed of different elements derived from different native promoters or synthetic DNA segments. According to one embodiment, tissue-specific expression can be used and may be advantageous when expression of one or more polynucleotides in certain tissues is preferred. Examples of tissue-specific promoters under developmental control include promoters that can initiate transcription only (or primarily only) in certain tissues, such as vegetative tissues, for example, roots or leaves, or reproductive tissues, such as fruit, ovules, seeds, pollen, pistols, flowers, or any embryonic tissue. Reproductive tissue-specific promoters may be, for example, anther-specific, ovule-specific, embryo-specific, endosperm-specific, integument-specific, seed and seed coat-specific, pollen-specific, petal-specific, sepal-specific, or combinations thereof. Suitable leaf-specific promoters include pyruvate, orthophosphate dikinase (PPDK) promoter from C4 plant (maize), cab-m1Ca+2 promoter from maize, the Arabidopsis thaliana myb-related gene promoter (Atmyb5), the ribulose biphosphate carboxylase (RBCS) promoters (for example, the tomato RBCS 1, RBCS2 and RBCS3A genes expressed in leaves and light-grown seedlings, RBCS1 and RBCS2 expressed in developing tomato fruits or ribulose bisphosphate carboxylase promoter expressed almost exclusively in mesophyll cells in leaf blades and leaf sheaths at high levels). Suitable senescence-specific promoters include a tomato promoter active during fruit ripening, senescence and abscission of leaves, a maize promoter of gene encoding a cysteine protease. Suitable anther-specific promoters can be used. Suitable root-preferred promoters known to persons skilled in the art may be selected. Suitable seed-preferred promoters include both seed-specific promoters (those promoters active during seed development such as promoters of seed storage proteins) and seed-germinating promoters (those promoters active during seed germination). Such seed-preferred promoters include, but are not limited to, Cim1 (cytokinin-induced message); milps (myo-inositol-1-phosphate synthase); mZE40-2, also known as Zm-40; nuclc; and celA (cellulose synthase). Glob-1 is an embryo-specific promoter. For dicots, seed-specific promoters include, but are not limited to, bean beta-phaseolin, napin, beta-conglycinin, soybean lectin, cruciferin, and the like. For monocots, seed-specific promoters include, but are not limited to, a maize 15 kDa zein promoter, a 22 kDa zein promoter, a 27 kDa zein promoter, a g-zein promoter, a 27 kDa gamma-zein promoter (such as gzw64A promoter, see Genbank Accession #S78780), a waxy promoter, a shrunken 1 promoter, a shrunken 2 promoter, a globulin 1 promoter (see Genbank Accession # L22344), an Ltp2 promoter, cim1 promoter, maize end1 and end2 promoters, nuc1 promoter, Zm40 promoter, eep1 and eep2; lec1, thioredoxin H promoter; mlip15 promoter, PCNA2 promoter; and the shrunken-2 promoter. Examples of inducible promoters include promoters responsive to pathogen attack, anaerobic conditions, elevated temperature, light, drought, cold temperature, or high salt concentration. Pathogen-inducible promoters include those from pathogenesis-related proteins (PR proteins), which are induced following infection by a pathogen (for example, PR proteins, SAR proteins, beta-1,3-glucanase, chitinase).

In addition to plant promoters, other suitable promoters may be derived from bacterial origin for example, the octopine synthase promoter, the nopaline synthase promoter and other promoters derived from Ti plasmids), or may be derived from viral promoters (for example, 35S and 19S RNA promoters of cauliflower mosaic virus (CaMV), constitutive promoters of tobacco mosaic virus, cauliflower mosaic virus (CaMV) 19S and 35S promoters, or figwort mosaic virus 35S promoter). The regulatory sequence may also contain a transcription termination sequence that is functional in a plant. The regulatory sequence may also contain a translation enhancer functional in plant. An enhancer is a nucleic acid sequence that can recruit transcriptional regulatory proteins such as transcriptional activators, to enhance transcriptional activation by increasing promoter activity. Suitable enhancers can be derived from regions proximate to a native promoter of interest (homologous sources) or can be derived from non-native contexts (heterologous sources) and operably-linked to any promoter of interest to enhance the activity or the tissue-specificity of a promoter. Some enhancers can operate in any orientation with respect to the orientation of a transcription unit. For example, enhancers may be positioned upstream or downstream of a transcriptional unit comprising a promoter and a nucleic acid construct.

The plant host cell used for the expression of recombinant Apolipoprotein may be derived or derivable from a plant or it may be a cultured plant cell that is cultured outside of a plant. Thus, in one embodiment, the plant is a plant cell - such as a plant cell grown in culture or outside of a plant such as an in vitro grown plant cell or clumps of cells. Non-limiting examples of plants include monocots and dicots, such as crop plants, ornamental plants, and non-domesticated or wild plants. Further examples include plants of commercial or agricultural interest, such as crop plants (especially crop plants used for human food or animal feed), wood- or pulp-producing trees, vegetable plants, fruit plants, and ornamental plants.

Techniques for introducing (for example, transforming or infiltrating) one or more nucleic acid molecules into a plant - such as monocotyledonous and dicotyledonous plants - are known in the art. Any method may be used to introduce the nucleic acid molecule(s), vectors, constructs and the like into a plant. By way of example, they may be introduced into a plant by biolistics or gene gun techniques employing microparticles coated with the construct(s) Agrobacterium-mediated transformation (for example, using *A. radiobacter*, A. rhizogenes, *A. rubi*, or *A. tumefaciens*), viral vector-mediated transformation, electroporation and infiltration by Agrobacterium cells, also referred to as agroinfiltration. In one embodiment, Agrobacterium-mediated transformation of plant cells is used. In another embodiment, agroinfiltration is used to introduced the nucleic acids into a whole plant, an intact plant, or a part thereof. Agroinfiltration can be carried out under reduced air pressure or a vacuum by techniques and apparatus known in the art.

The introduction of a nucleic acid into a plant may give rise to stable expression of the protein encoded by the nucleic acid. Typically, stable expression will result in the integration of the nucleic acid into the host genome so as to create a transgenic plant and the nucleic acid will be passed onto the next generation. The introduction of a nucleic acid into a plant may give rise to transient expression of the protein encoded by the nucleic acid. Transient expression does not necessarily rely on the integration of the nucleic acid into the host genome. Typically, tobacco plants infiltrated with Agrobacterium cells are incubated for 5, 10, 15, or 20 days or more before the plant parts are harvested to recover the recombinantly produced protein. Both forms of expression are contemplated by the present invention.

The plants into which the nucleic acid has been introduced can be incubated and progeny obtained optionally under selection if a selectable marker gene is employed,. These progeny may be used to prepare transgenic seeds, or alternatively, bred with a another plant. The seeds obtained from such progeny may be germinated, cultivated, and used to prepare subsequent generations of offspring which comprise the nucleic acid originally introduced. An immature embryo or embryogenic calli from a plant may be used as a starting material. These techniques are routine and well known to one of ordinary skill in the art. Once the plant matures then the tissue into which the nucleotide sequence is expressed is harvested and recovered therefrom using the methods described herein. In some embodiments, the method of introducing the nucleic acid into a plant may make the plants less healthy in which case it may be desirable to incubate the plants under conditions to try and prolong the survival thereof. According to some embodiments, it may desirable to harvest the plant tissue after 5, 10, 15, or 20 days or more after the introduction of nucleic acid.

The nucleic acid sequence, nucleic acid construct, or vector and the like comprises, in a further embodiment, a nucleic acid sequence encoding a suppressor of gene silencing of, for example, viral origin. In one embodiment, the suppressor of gene silencing is that of a virus selected from the group consisting of Havel river virus (HaRV), Pear latent virus (PeLV), Lisianthus necrosis virus, Grapevine Algerian latent virus, Pelargonium necrotic spot virus (PeNSV), Cymbidium ringspot virus (CymRSV), Artichoke mottled crinkle virus (AMCV), Carnation Italian ringspot virus (CIRV), Lettuce necrotic stunt virus, rice yellow mottle virus (RYMV), potato virus X (PVX), African cassava mosaic virus (ACMV), Cucumber mosaic virus (CMV), Cucumber necrosis virus (CNV), potato virus Y (PVY), tobacco etch virus (TEV), and Tomato bushy stunt virus (TBSV) or a combination of two or more thereof. Examples of suppressor of gene silencing that can be used in the invention include but are not limited to the p1 protein of rice yellow mottle virus (RYMV), the p25 protein of potato virus X (PVX), the AC2 protein of African cassava mosaic virus (ACMV), the 2b protein of cucumber mosaic virus (CMV), the 19 kDa p19 protein of Cucumber necrosis virus (CNV), the helper-component proteinase (HcPro) of potato virus Y (PVY), tobacco etch virus (TEV) and Tomato bushy stunt virus (TBSV) In one embodiment, the suppressor of gene silencing is HcPro of tobacco etch virus (TEV). In another embodiment, the suppressor of gene silencing is the p19 protein of Tomato bushy stunt virus (TBSV). Accordingly, in a further embodiment, there is provided a nucleic acid construct comprising: a first nucleic acid sequence encoding a protein that induces the formation of a protein body in a plant, optionally, further comprising a nucleic acid sequence encoding a non-naturally occurring repeat sequence motif; a second nucleic acid sequence encoding Apolipoprotein; and optionally a third nucleic acid sequence encoding an amino acid linker in which a peptide bond therein can be specifically cleaved; wherein said first, second and third nucleic acid sequences are operably linked to each other and optionally, a regulatory nucleotide sequence that regulates the transcription of said nucleic acid sequence(s) and optionally an expressible nucleic acid encoding a suppressor of gene silencing, suitably of viral origin. In an alternative embodiment, the expressible nucleic acid encoding a suppressor of gene silencing can be a separate second nucleic acid molecule or a part of a second nucleic acid which is introduced to the plant or plant cells, and coexpressed in the plant or plant cells that are also producing Apolipoprotein.

For example, stable plant transformation can be carried out as follows: vectors are transferred into *Agrobacterium tumefaciens*. Tobacco (*Nicotiana benthamiana or N. tabacum*) leaf discs are transformed according to the method of Draper et al. (1988) In: Plant Genetic Transformation and Gene Expression. A Laboratory Manual (Eds. Draper, J. , Scott, R., Armitage, P. and Walden, R.), Blackwell Scientific Publications. Regenerated plants are selected on medium comprising 200 mg/L kanamycin and transferred to a greenhouse. Transformed tobacco plants having the highest transgene product levels are cultivated to obtain a T1 generation. Developing leaves (approximately 12 cm long) are harvested, immediately frozen with liquid nitrogen and stored at-80°C for further experiments.

The plant host cell may be a grain crop plants (such as wheat, oat, barley, maize, rye, triticale, rice, millet, sorghum, quinoa, amaranth, and buckwheat); forage crop plants (such as forage grasses and forage dicots including alfalfa, vetch, clover, and the like); oilseed crop plants (such as cotton, safflower, sunflower, soybean, canola, rapeseed, flax, peanuts, and oil palm); tree nuts (such as walnut, cashew, hazelnut, pecan, almond, and the like); sugarcane, coconut, date palm, olive, sugarbeet, tea, and coffee; wood- or pulp-producing trees; vegetable crop plants such as legumes (for example, beans, peas, lentils, alfalfa, peanut), lettuce, asparagus, artichoke, celery, carrot, radish, the brassicas (for example, cabbages, kales, mustards, and other leafy brassicas, broccoli, cauliflower, Brussels sprouts, turnip, kohlrabi), cucurbits (for example, cucumbers, melons, summer squashes, winter squashes), alliums (for example, onions, garlic, leeks, shallots, chives), members of the Solanaceae (for example, tomatoes, eggplants, potatoes, peppers, groundcherries), and members of the Chenopodiaceae (for example, beet, chard, spinach, quinoa, amaranth); fruit crop plants such as apple, pear, citrus fruits (for example, orange, lime, lemon, grapefruit, and others), stone fruits (for example, apricot, peach, plum, nectarine), banana, pineapple, grape, kiwifruit, papaya, avocado, and berries; and ornamental plants including ornamental flowering plants, ornamental trees and shrubs, ornamental groundcovers, and ornamental grasses. Further examples of dicot plants include, but are not limited to, canola, cotton, potato, quinoa, amaranth, buckwheat, safflower, soybean, sugarbeet, and sunflower, more suitably soybean, canola, and cotton. Further examples of monocots include, but are not limited to, wheat, oat, barley, maize, rye, triticale, rice, ornamental and forage grasses, sorghum, millet, and sugarcane.

The plant host cell may be or may be derived from a monocotyledonous or dicotyledonous plant or a plant cell system, including species from one of the following families: Acanthaceae, Alliaceae, Alstroemeriaceae, Amaryllidaceae, Apocynaceae, Arecaceae, Asteraceae, Berberidaceae, Bixaceae, Brassicaceae, Bromeliaceae, Cannabaceae, Caryophyllaceae, Cephalotaxaceae, Chenopodiaceae, Colchicaceae, Cucurbitaceae, Dioscoreaceae, Ephedraceae, Erythroxylaceae, Euphorbiaceae, Fabaceae, Lamiaceae, Linaceae, Lycopodiaceae, Malvaceae, Melanthiaceae, Musaceae, Myrtaceae, Nyssaceae, Papaveraceae, Pinaceae, Plantaginaceae, Poaceae, Rosaceae, Rubiaceae, Salicaceae, Sapindaceae, Solanaceae, Taxaceae, Theaceae, or Vitaceae.

Suitable species may include members of the genera Abelmoschus, Abies, Acer, Agrostis, Allium, Alstroemeria, Ananas, Andrographis, Andropogon, ArteApolipoproteinia, Arundo, Atropa, Berberis, Beta, Bixa, Brassica, Calendula, Camellia, Camptotheca, Cannabis, Capsicum, Carthamus, Catharanthus, Cephalotaxus, Chrysanthemum, Cinchona, Citrullus, Coffea, Colchicum, Coleus, CucuApolipoprotein, Cucurbita, Cynodon, Datura, Dianthus, Digitalis, Dioscorea, Elaeis, Ephedra, Erianthus, Erythroxylum, Eucalyptus, Festuca, Fragaria, Galanthus, Glycine, Gossypium, Helianthus, Hevea, Hordeum, Hyoscyamus, Jatropha, Lactuca, Linum, Lolium, Lupinus, Lycopersicon, Lycopodium, Manihot, Medicago, Mentha, Apolipoproteincanthus, Musa, Nicotiana, Oryza, Panicum, Papaver, Parthenium, Pennisetum, Petunia, Phalaris, Phleum, Pinus, Poa, Poinsettia, Populus, Rauwolfia, Ricinus, Rosa, Saccharum, Salix, Sanguinaria, Scopolia, Secale, Solanum, Sorghum, Spartina, Spinacea, Tanacetum, Taxus, Theobroma, Triticosecale, Triticum, Uniola, Veratrum, Vinca, Vitis, and Zea.

Other suitable species may include Panicum spp., Sorghum spp., Apolipoproteincanthus spp., Saccharum spp., Erianthus spp., Populus spp., Andropogon gerardii (big bluestem), Pennisetum purpureum (elephant grass), Phalaris arundinacea (reed canarygrass), Cynodon dactylon (bermudagrass), Festuca arundinacea (tall fescue), Spartina pectinata (prairie cord-grass), Medicago sativa (alfalfa), Arundo donax (giant reed), Secale cereale (rye), Salix spp. (willow), Eucalyptus spp. (eucalyptus), Triticosecale (triticum--wheat.times.rye), bamboo, Helianthus annuus (sunflower), Carthamus tinctorius (safflower), Jatropha curcas (jatropha), Ricinus communis (castor), Elaeis guineensis (palm), Linum usitatissimum (flax), Brassica juncea, Beta vulgaris (sugarbeet), Manihot esculenta (cassaya), Lycopersicon esculentum (tomato), Lactuca sativa (lettuce), Musa paradisiaca (banana), Solanum tuberosum (potato), Brassica oleracea (broccoli, cauliflower, Brussels sprouts), Camellia sinensis (tea), Fragaria ananassa (strawberry), Theobroma cacao (cocoa), Coffea arabica (coffee), Vitis vinifera (grape), Ananas comosus (pineapple), Capsicum annum (hot & sweet pepper), Allium cepa (onion), CucuApolipoprotein melo (melon), CucuApolipoprotein sativus (cucumber), Cucurbita maxima (squash), Cucurbita moschata (squash), Spinacea oleracea (spinach), Citrullus lanatus (watermelon), Abelmoschus esculentus (okra), Solanum melongena (eggplant), Rosa spp. (rose), Dianthus caryophyllus (carnation), Petunia spp. (petunia), Poinsettia pulcherrima (poinsettia), Lupinus albus (lupin), Uniola paniculata (oats), bentgrass (Agrostis spp.), Populus tremuloides (aspen), Pinus spp. (pine), Abies spp. (fir), Acer spp. (maple), Hordeum vulgare (barley), Poa pratensis (bluegrass), Lolium spp. (ryegrass) and Phleum pratense (timothy), Panicum virgatum (switchgrass), Sorghum bicolor (sorghum, sudangrass), Apolipoproteincanthus giganteus (Apolipoproteincanthus), Saccharum sp. (energycane), Populus balsamifera (poplar), Zea mays (corn), Glycine max (soybean), Brassica napus (canola), Triticum aestivum (wheat), Gossypium hirsutum (cotton), Oryza sativa (rice), Helianthus annuus (sunflower), Medicago sativa (alfalfa), Beta vulgaris (sugarbeet), or Pennisetum glaucum (pearl millet).

In a particularly suitable embodiment, the plant host cell may be or may be derived from a naturally occurring, a mutant, a non-naturally occurring or a transgenic tobacco plant. A tobacco plant includes plants of the genus *Nicotiana*, various species of *Nicotiana*, including *N. rustica* and *N. tabacum*. Other species include *N. acaulis, N. acuminata, N. acuminata var. multiflora, N. africana, N. alata, N. amplexicaulis, N. arentsii, N. attenuata, N. benavidesii, N. benthamiana, N. bigelovii, N. bonariensis, N. cavicola, N. clevelandii, N. cordifolia, N. corymbosa, N. debneyi, N. excelsior, N. forgetiana, N. fragrans, N. glauca, N. glutinosa, N. goodspeedii, N. gossei, N. hybrid, N. ingulba, N. kawakamii, N. knightiana, N. langsdorffii, N. linearis, N. longiflora, N. maritima, N. megalosiphon, N. miersii, N. noctiflora, N. nudicaulis, N. obtusifolia, N. occidentalis, N. occidentalis subsp. hesperis, N. otophora, N. paniculata, N. pauciflora, N. petunioides, N. plumbaginifolia, N. quadrivalvis, N. raimondii, N. repanda, N. rosulata, N. rosulata subsp. ingulba, N. rotundifolia, N. setchellii, N. simulans, N. solanifolia, N. spegazzinii, N. stocktonii, N. suaveolens, N. sylvestris, N. tabacum. N. thyrsiflora, N. tomentosa, N. tomentosiforApolipoprotein, N. trigonophylla, N. umbratica, N. undulata, N. velutina, N. wigandioides, and N. x sanderae*.

The use of a plant host cell that is or is derived from cultivars or elite cultivars is also contemplated. Non-limiting examples of varieties or cultivars are: BD 64, CC 101, CC 200, CC 27, CC 301, CC 400, CC 500, CC 600, CC 700, CC 800, CC 900, Coker 176, Coker 319, Coker 371 Gold, Coker 48, CD 263, Denzizli, DF911, Galpao tobacco, GL 26H, GL 350, GL 600, GL 737, GL 939, GL 973, HB 04P, K 149, K 326, K 346, K 358, K394, K 399, K 730, KDH 959, KT 200, KT204LC, KY10, KY14, KY 160, KY 17, KY 171, KY 907, KY907LC, KTY14xL8 LC, Karabaglar, Little Crittenden, McNair 373, McNair 944, msKY 14xL8, Narrow Leaf Madole, NC 100, NC 102, NC 2000, NC 291, NC 297, NC 299, NC 3, NC 4, NC 5, NC 6, NC7, NC 606, NC 71, NC 72, NC 810, NC BH 129, NC 2002, Neal Smith Madole, OXFORD 207, 'Perique' tobacco, PVH03, PVH09, PVH19, PVH50, PVH51, R 610, R 630, R 7-11, R 7-12, RG 17, RG 81, RG H51, RGH 4, RGH 51, RS 1410, Speight 168, Speight 172, Speight 179, Speight 210, Speight 220, Speight 225, Speight 227, Speight 234, Speight G-28, Speight G-70, Speight H-6, Speight H20, Speight NF3, TI 1406, TI 1269, TN 86, TN86LC, TN 90, TN 97, TN97LC, TN D94, TN D950, TR (Tom Rosson) Madole, Turkish Samson, VA 309, VA359, DAC, Mata, Fina, PO2, BY-64, AS44, RG17, RG8, HB04P, Basma Xanthi BX 2A, Coker 319, Hicks, McNair 944 (MN 944), Burley 21, K149, Yaka JB 125/3, Kasturi Mawar, NC 297, Coker 371 Gold, PO2, Wislica, Simmaba, Turkish Samsun, AA37-1, B13P, F4 from the cross BU21 x Hoja Parado line 97, Samsun, PO1, LA B21, LN KY171, TI 1406, Basma, Galpao, Perique, Beinhart 1000-1 or Petico. Non-limiting examples of N. tabacum cultivars are AA 37-1, B 13P, Xanthi (Mitchell-Mor), KTRD#3 Hybrid 107, Bel-W3, 79-615, Samsun Holmes NN, KTRDC#2 Hybrid 49, KTRDC#4 Hybrid 110, Burley 21, BY-64, KTRDC#5 KY 160 SI, KTRDC#7 FCA, KTRDC#6 TN 86 SI, Coker 371 Gold, K 149, K 326, K 346, K 358, K 394, K 399, K 730, KY 10, KY 14, KY 160, KY 17, KY 8959, KY 9, KY 907, MD 609, McNair 373, NC 2000, PG 01, PG 04, M066, PO1, PO2, PO3, RG 11, RG 17, RG 8, Speight G-28, TN 86, TN 90, VA 509, AS44, Banket A1, Basma Drama B84/31, Basma I Zichna ZP4/B, Basma Xanthi BX 2A, Batek, Besuki Jember, C104, Coker 319, Coker 347, Criollo Apolipoproteinionero, DAC Mata Fina, Delcrest, Djebel 81, DVH 405, Galpão Comum, HB04P, Hicks Broadleaf, Kabakulak Elassona, Kasturi Mawar, Kutsage E1, KY 14xL8, KY 171, LA BU 21, McNair 944, NC 2326, NC 71, NC 297, NC 3, PVH 03, PVH 09, PVH 19, PVH 2110, Red Russian, Samsun, Saplak, Simmaba, Talgar 28, Turkish Samsun, Wislica, Yayaldag, NC 4, TR Madole, Prilep HC-72, Prilep P23, Prilep PB 156/1, Prilep P12-2/1, Yaka JK-48, Yaka JB 125/3, TI-1068, KDH-960, TI-1070, TW136, Samsun NN, Izmir, Basma, TKF 4028, L8, TKF 2002, TN90, GR141, Basma xanthi, GR149, GR153, Petit Havana or Xanthi NN.

The plant host cell may be modified to improve the expression and/or activity of the recombinant Apolipoprotein. The host cell may, for example, be modified to include chaperone proteins that further promote the formation of Apolipoprotein. The host cell may be modified to include a repressor protein to more efficiently regulate the expression of Apolipoprotein or even an enhancer protein to improve expression levels.

The method for producing Apolipoprotein in a plant comprises the second step of growing said plant under conditions that allow for the expression of Apolipoprotein as a fusion protein in said plant. Accordingly, the Apolipoprotein polypeptide is prepared by culturing transformed plant cells under culture conditions suitable to express the polypeptide as a fusion protein. The resulting polypeptide is expressed in the endoplasmic reticulum of a plant cell in the form of protein bodies.

Apolipoprotein expression may be measured by detecting the amount of mRNA encoding an Apolipoprotein polypeptide in the cell which can be quantified by, for example, PCR or Northern blot. Where a change in the amount of Apolipoprotein polypeptide in the sample is being measured, detecting Apolipoprotein by use of anti-Apolipoprotein antibodies can be used to quantify the amount of Apolipoprotein polypeptide in the cell using known techniques. Alternatively the biological activity of Apolipoprotein can be measured.

Various methods may be utilised to recover the protein bodies comprising the fusion protein. The recombinant protein body-like assemblies have a density that can be predetermined for a particular fusion protein. The predetermined density is typically greater than that of substantially all of the endogenous host cell proteins present in the homogenate, and is typically about 1.1 to about 1.35 g/ml. The high density of the protein bodies may be due to the general ability of the recombinant fusion proteins to assemble as multimers and accumulate. When expressed in plants, the protein bodies are typically spherical in shape with diameters of about 1 micron and have a surrounding membrane.

Recovery of the protein bodies by density is typically carried out using a centrifuge. The centrifugation may be carried out in the presence of a differential density-providing solute - such as a salt (for example, caesium chloride) or a sugar (for example, sucrose). Regions of different density may be formed in the homogenate to provide a region that contains a relatively enhanced concentration of the protein bodies and a region that contains a relatively depleted concentration of the protein bodies. The protein body-depleted region may be separated from the region of relatively enhanced concentration of protein bodies, thereby recovering said fusion protein. The protein bodies can be collected or can be treated with one or more reagents or subjected to one or more procedures prior to isolation of the protein bodies comprising the fusion protein, as described herein. In some embodiments, the collected protein bodies are used as is, without the need to isolate the fusion protein.

In some embodiments, one low speed centrifugation step may be sufficient to recover the protein bodies in the form of a pellet. Thus, by way of example, centrifugation at 200 x g for 10 minutes at 4°C may be sufficient. In other embodiments, more than one centrifugation step may be performed in which the low speed centrifugation step is combined with one or more higher speed centrifugation steps. Thus, by way of example, a centrifugation step of about 200 x g for 10 minutes at 4°C to remove solids and cell debris may be combined with a higher speed centrifugation step of, for example, about 6000 x g for 10 minutes at 4°C to recover the fusion protein in the pellet.

This centrifugation step may optionally be followed by one or wash steps in a solution comprising a surfactant (for example, a non-ionic surfactant) together with a further optional centrifugation step to concentrate and enrich the protein bodies prior to solubilisation thereof. The further optional centrifugation step may be carried out between washes. In one embodiment, the surfactant used is Triton X-100, suitably 1% Triton X-100. In another embodiment, the further centrifugation step is carried out at about 1500 x g for 10 minutes at 4°C which may occur between washes.

Thus, according to one embodiment of the invention, the method comprises the additional step of: (c) recovering the protein body comprising the fusion protein from the plant or plant material, preferably wherein step (c) comprises the steps of: (i) homogenising the plant material; (ii) centrifuging the homogenised plant material at low speed, preferably, about 200 x g; (iii) centrifuging the homogenised plant material at a higher speed than step (ii), preferably, about 6000 x g; and (iv) recovering the protein bodies comprising the fusion protein in the pelleted fraction.

In order to solubilise the fusion protein contained in the pelleted fraction, various buffers and reagents may be used. By way of example, the fusion protein comprising Apolipoprotein may be obtained from the collected protein bodies by dissolution of the surrounding membrane in an aqueous buffer comprising a detergent and/or a reducing agent. Examples of reducing agents include 2-mercaptoethanol, thioglycolic acid, thioglycolate salts, dithiothreitol (DTT), sulfite or bisulfite ions. Examples of detergents include sodium dodecyl sulfate (SDS), ionic detergents (for example, deoxycholate and lauroylsarcosine), non-ionic detergents (for example, Tween 20, Nonidet P-40 and octyl glucoside) and zwitterionic detergents (for example, CHAPS). Conditions are chosen so as to not disrupt and unfold the attached Apolipoprotein.

The variables that can be tested in order to identify appropriate solubilisation conditions include pH, salt, detergent, reducing agent, as well as other variables such as ratio of components, time and temperature. In one embodiment, solubilisation can be achieved using a buffer comprising urea, dithiothreitol and tris(2-carboxyethyl)phosphine), suitably at a ratio of protein bodies:buffer of 1:10 (w/v). The protein bodies may be incubated with the buffer overnight at room temperature and/or together with a cell disrupter. Various buffers can be employed depending on the desired pH of the buffer. Non-limiting examples of buffer components that can be used to control the pH range include acet ate, citrate, histidine, phosphate, ammonium buffers such as ammonium acetate, succinate, MES, CHAPS, MOPS, MOPSO, HEPES, Tris, and the like, as well as combinations of these TRIS-malic acid-NaOH, maleate, chloroacetate, formate, benzoate, propionate, pyridine, piperazine, ADA, PIPES, ACES, BES, TES, tricine, bicine, TAPS, ethanolamine, CHES, CAPS, methylamine, piperidine, boric acid, carbonic acid, lactic acid, butaneandioic acid, diethylmalonic acid, glycylglycine, HEPPS, HEPPSO, imidazole, phenol, POPSO, succinate, TAPS, amine-based, benzylamine, trimethyl or dimethyl or ethyl or phenyl amine, ethylenediamine, or mopholine. In one embodiment, the buffer has a pH of about 8. In another embodiment, the buffer is 50mM Tris pH 9 comprising a reducing agent, preferably, beta-mercaptoethanol, a non-ionic surfactant, preferably, Triton X-100 and optionally salt, preferably, sodium chloride. In another embodiment, the buffer comprises about 50mM Tris pH9 comprising about 20 mM beta-mercaptoethanol, about 1% Triton X-100 and about 500 mM sodium chloride.

Accordingly, the method of the present invention may comprise the further step of: (d) solubilising the fusion protein, preferably, wherein said solubilisation step comprises the use of a mixture comprising, consisting or consisting essentially of a reducing agent, a non-ionic surfactant and optionally salt. Optionally, the preparation may be centrifuged prior to the next method step, for example at about 16000 x g at 4°C for 10 minutes.

The separated, solubilised fusion protein that comprises the Apolipoprotein protein is collected. At this stage, the Apolipoprotein protein may be used as is. Preferably, the Apolipoprotein protein is further processed.

Accordingly, in one embodiment, the method comprises the further step of releasing Apolipoprotein from said fusion protein. The cleavage of Apolipoprotein from the fusion protein is described herein.

Following cleavage, in a further embodiment, the method comprises the additional step of: (f) purifying the cleaved/released Apolipoprotein. Thus, in one embodiment, the recombinant Apolipoprotein thus purified is substantially free of other polypeptides as determined by, for example, SDS-PAGE or ELISA. In another embodiment, purified Apolipoprotein is considered to be a Apolipoprotein composition which contains less than 100 ppm host protein and suitably less than 90 ppm, less than 80 ppm, less than 70 ppm, less than 60 ppm, less than 50 ppm, less than 40 ppm, less than 30 ppm, less than 20 ppm, less than 10 ppm, or less than 5 ppm host protein, as determined by, for example, SDS-PAGE or ELISA.. The Apolipoprotein obtained or obtainable according to the present invention can have a specific activity of at least 50%, 60%, or 70%, and most suitably at least 80%, 90%, 95% or 100% that of the native protein that the sequence is derived from.

Protein purification may utilise a "cation exchange resin" which is negatively charged, and which has free cations for exchange with cations in an aqueous solution passed over or through the adsorbent or solid phase. Any negatively charged ligand suitable to form the cation exchange resin can be used, for example, a carboxylate, sulfonate and others as described below. Commercially available cation exchange resins include, but are not limited to, for example, those having a sulfonate based group (for example, MonoS, MiniS, Source 15S and 3OS, SP Sepharose Fast Flow™,SP Sepharose High Performance from GE Healthcare, Toyopearl SP-650S and SP-650M from Tosoh, Macro-Prep High S from BioRad, Ceramic HyperD S, Trisacryl M and LS SP and Spherodex LS SP from Pall Technologies); a sulfoethyl based group (for example, Fractogel SE, from EMD, Poros S- 10 and S-20 from Applied Biosystems); a sulphopropyl based group (for example, TSK Gel SP 5PW and SP-5PW-HR from Tosoh, Poros HS-20 and HS 50 from Applied Biosystems); a sulfoisobutyl based group (for example, (Fractogel EMD SO₃^{"} from EMD); a sulfoxyethyl based group (for example, SE52, SE53 and Express-Ion S from Whatman), a carboxymethyl based group (for example, CM Sepharose Fast Flow from GE Healthcare, Hydrocell CM from Biochrom Labs Inc., Macro-Prep CM from BioRad, Ceramic HyperD CM, Trisacryl M CM, Trisacryl LS CM, from Pall Technologies, Matrx Cellufme C500 and C200 from Millipore, CM52, CM32, CM23 and Express -Ion C from Whatman, Toyopearl CM- 650S, CM-650M and CM-650C from Tosoh); sulfonic and carboxylic acid based groups (for example BAKEPVBOND Carboxy-Sulfon from J.T. Baker); a carboxylic acid based group (for example, WP CBX from J.T Baker, DOWEX MAC-3 from Dow Liquid Separations, Amberlite Weak Cation Exchangers, DOWEX Weak Cation Exchanger, and Diaion Weak Cation Exchangers from Sigma-Aldrich and Fractogel EMD COO- from EMD); a sulfonic acid based group (e. g., Hydrocell SP from Biochrom Labs Inc., DOWEX Fine Mesh Strong Acid Cation Resin from Dow Liquid Separations, UNOsphere S, WP Sulfonic from J. T. Baker, Sartobind S membrane from Sartorius, Amberlite Strong Cation Exchangers, DOWEX Strong Cation and Diaion Strong Cation Exchanger from Sigma-Aldrich); and a orthophosphate based group (for example, PI 1 from Whatman).

Protein purification may utilise an "anion exchange resin" which is positively charged, thus having one or more positively charged ligands attached thereto. Any positively charged ligand attached to the adsorbent or solid phase suitable to form the anionic exchange resin can be used, such as quaternary amino groups Commercially available anion exchange resins include DEAE cellulose, Poros PI 20, PI 50, HQ 10, HQ 20, HQ 50, D 50 from Applied Biosystems, Sartobind Q from Sartorius, MonoQ, MiniQ, Source 15Q and 3OQ, Q, DEAE and ANX Sepharose Fast Flow, Q Sepharose high Performance, QAE SEPHADEX™ and FAST Q SEPHAROSE™ (GE Healthcare), WP PEI, WP DEAM, WP QUAT from J.T. Baker, Hydrocell DEAE and Hydrocell QA from Biochrom Labs Inc., UNOsphere Q, Macro-Prep DEAE and Macro-Prep High Q from Biorad, Ceramic HyperD Q, ceramic HyperD DEAE, Trisacryl M and LS DEAE, Spherodex LS DEAE, QMA Spherosil LS, QMA Spherosil M and Mustang Q from Pall Technologies, DOWEX Fine Mesh Strong Base Type I and Type II Anion Resins and DOWEX MONOSPHER E 77, weak base anion from Dow Liquid Separations, Intercept Q membrane, Matrex Cellufme A200, A500, Q500, and Q800, from Millipore, Fractogel EMD TMAE, Fractogel EMD DEAE and Fractogel EMD DMAE from EMD, Amberiite weak strong anion exchangers type I and II, DOWEX weak and strong anion exchangers type I and II, Diaion weak and strong anion exchangers type I and II, Duolite from Sigma-Aldrich, TSK gel Q and DEAE 5PW and 5PW-HR, Toyopearl SuperQ-650S, 650M and 650C, QAE-550C and 650S, DEAE-650M and 650C from Tosoh, QA52, DE23, DE32, DE51, DE52, DE53, Express-Ion D and Express-Ion Q from Whatman.

"Affinity chromatography" is another method of protein purification which refers to a separation technique in which a protein is reversibly and specifically bound to a biologically specific ligand, usually as a combination of spatial complementarity and one or more types of chemical interactions, for example, electrostatic forces, hydrogen bonding, hydrophobic forces, and van der Waals forces at the binding site. These interactions are not due to the general properties of the molecule such as isoelectric point, hydrophobicity or size but are a result of specific interactions between the protein and the ligand, for example, immunoglobulin binding to an epitope, protein A binding to immunoglobulin, interactions between a biological response modifier and its cell surface receptor. In many instances, the biologically specific ligand is also a protein or a polypeptide and can be immobilized onto a solid phase, such as the bead.

A "mixed mode ion exchange resin" is another method of protein purification and refers to a solid phase which is covalently modified with cationic, anionic or hydrophobic moieties. Examples of mixed mode ion exchange resins include BAKERBOND ABX™ (J. T. Baker; Phillipsburg, NJ), ceramic hydroxyapatite type I and II and fluoride hydroxyapatite (BioRad; Hercules, CA) and MEP and MBI HyperCel (Pall Corporation; East Hills, NY). Hydrophobic charge induction chromatography (or "HClC") is a type of mixed mode chromatographic process in which the protein in the mixture binds to an ionizable ligand through mild hydrophobic interactions in the absence of added salts (for example, a lyotropic salts). The mixed mode refers to one mode for binding and another mode for elution, For example, a solid phase useful in HClC contains a ligand which has the combined properties of thiophilic effect (i.e., utilizing the properties of thiophilic chromatography), hydrophobicity and an ionizable group for its separation capability. Accordingly, an adsorbent used in a method of the invention contains a ligand that is ionizable and mildly hydrophobic at neutral (physiological) or slightly acidic pH, for example, about pH 5 to 10, preferably about pH 6 to 9.5. At this pH range, the ligand is predominantly uncharged and binds a protein via mild non-specific hydrophobic interaction. As pH is reduced, the ligand acquires charge and hydrophobic binding is disrupted by electrostatic charge repulsion towards the solute due to the pH shift. Examples of suitable ligands for use in HClC include any ionizable aromatic or heterocyclic structure (for example, those having a pyridine structure, such as 2- aminomethylpyridine, 3-aminomethylpyridine and 4-aminomethylpyridine, 2- mercaptopyridine, 4-mercaptopyridine or 4-mercaptoethylpyridine, mercaptoacids, mercaptoalcohols, imidazolyl based, mercaptomethylimidazole, 2-mercaptobenzimidazole, aminomethylbenzimidazole, histamine, mercaptobenz imidazole, diethylammopropylamine, aminopropyhnorpholine, aminopropylimidazole, aminocaproic acid, nitrohydroxybenzoic acid, nitrotyrosine/ethanolamine, dichlorosalicylic acid, dibromotyramine, chlorohydroxyphenylacetic acid, hydroxyphenylacetic acid, tyramine, thiophenol, glutathione, bisulphate, and dyes, including derivatives thereto.

In one embodiment, immobilized metal ion affinity chromatography is used. This is a separation technique that is based on coordinate covalent binding between proteins and metal ions. Proteins have a wide variety of amino acids composition which, in effect, generates a range of different affinities towards metal ions. Several different types of immobilized metal ion column have been developed to separate various proteins (for example, Fe, Co, Cd, Ni, or Zn). Thus, according to one embodiment, the prolamin, preferably, gamma-zein protein is immobilized to the metal ion column. According to another embodiment, an immobilized nickel column is used.

In another embodiment, reversed phase chromatography is used, which refers to a chromatographic method that uses a non-polar stationary phase. In another embodiment, reversed phase fast protein liquid chromatography is used.

In another embodiment, a combination of immobilized metal ion affinity chromatography and reversed phase chromatography are used. Suitably, immobilized metal ion affinity chromatography is used first which is followed by reversed phase chromatography are used.

Methods for purifying Apolipoprotein that are described in the art may also be used instead of the methods described herein or as an addition thereto. By way of example, WO9811140 describes a composition for use in a primary aqueous solution for purifying apolipoprotein comprising a first and a second polymeric material, said first and second polymeric material being immobilisible in the primary aqueous solution, and said second polymeric material being amphiphilic and water soluble. The method for purifying apolipoprotein comprises mixing the apolipoprotein, the composition and water, maintaining the resulting primary aqueous solution for a period of time sufficient for essentially separating the phases formed, removing the phase containing the second polymeric material and the main portion of Apolipoprotein, and thereafter separating the second polymeric material from Apolipoprotein.

In one embodiment, the method for producing Apolipoprotein in a plant comprises the steps of: (a) transforming a plant with a nucleic acid construct comprising, consisting or consisting essentially of a nucleic acid sequence encoding a prolamin protein, preferably gamma zein, that induces the formation of a protein body in a plant; and a nucleic acid sequence encoding Apolipoprotein, wherein said nucleic acid sequences are operably linked to each other; (b) growing said plant under conditions that allow for the expression of Apolipoprotein as a fusion protein in said plant; (c) recovering the protein body comprising the fusion protein from the plant; (d) solubilising the fusion protein; (e) releasing Apolipoprotein from said fusion protein; and (f) purifying the released Apolipoprotein.

A further aspect relates to a nucleic acid construct comprising said nucleic acid sequence and a regulatory nucleotide sequence that regulates the transcription of said nucleic acid sequence, as described herein. The construct may be a double-stranded, recombinant DNA fragment comprising one or more Apolipoprotein nucleic acids.

A further aspect relates to a vector comprising the nucleic acid sequence or the nucleic acid construct. Suitable vectors include, but are not limited to episomes capable of extra-chromosomal replication such as circular, double-stranded DNA plasmids; linearized double-stranded DNA plasmids; and other vectors of any origin. The vector includes a vector suitable for transforming bacteria and/or plants. The vector comprising the nucleic acid sequence or the construct described herein may be a plasmid, a cosmid or a plant vector that, when introduced into a cell, is integrated into the genome of said cell and is replicated along with the chromosome (or chromosomes) in which it has been integrated. A basic bacterial or plant vector suitably comprises a broad host range replication origin; a selectable marker; and, for Agrobacterium transformations, T-DNA sequences for Agrobacterium-mediated transfer to plant chromosomes. Sequences suitable for permitting integration of the heterologous sequences into the plant genome may be used as well. These might include transposon sequences, and the like, Cre/lox sequences and host genome fragments for homologous recombination, as well as Ti sequences which permit random insertion into a plant genome.

A promoter may be incorporated into the vector to create an expression vector which may be particularly useful for expressing the fusion proteins that are described herein. Suitable expression vectors include episomes capable of extra-chromosomal replication such as circular, double-stranded DNA plasmids; linearized double-stranded DNA plasmids; and other functionally equivalent expression vectors of any origin. An expression vector comprises at least a promoter operably-linked to an Apolipoprotein nucleic acid or an Apolipoprotein nucleic acid construct and the like. The promoter may be directly linked to the Apolipoprotein nucleic acid or there may be intervening nucleic acids in between - such as nucleic acids encoding one or more components of a fusion protein.

In preparing the nucleic acid sequences, nucleic acid constructs, nucleic acid vectors and the like, the various fragments thereof may be subjected to different processing conditions, such as ligation, restriction enzyme digestion, PCR, in vitro mutagenesis, linker and adapter addition, and the like. Thus, nucleotide transitions, transversions, insertions, deletions, or the like, may be performed on the DNA which is employed in the construct for expression of Apolipoprotein. Methods for restriction digests, Klenow blunt end treatments, ligations, and the like are well known to those in the art and are described, for example, by Maniatis et al. (in Molecular Cloning: A Laboratory Manual (1989) Cold Spring Harbor Laboratory Press, Cold Spring Harbor, N.Y.).

In another aspect, there is described a fusion protein comprising: (i) an amino acid sequence encoding a prolamin protein, preferably, gamma-zein that induces the formation of a protein body in a plant; (ii) an amino acid sequence encoding a cleavage recognition site; and (iii) an amino acid sequence encoding Apolipoprotein, wherein said first, second and third amino acid sequences are operably linked to each other. The fusion protein is the expression product of the nucleic acid sequence described herein in a plant cell. The fusion protein is accumulated in stable, endoplasmic reticulum-derived protein bodies in a plant cell.

In a further aspect, there is described a plant or plant material comprising the nucleic acid sequence, the nucleic acid construct, the vector or the fusion protein described herein.

In a further aspect, there is described Apolipoprotein obtained or obtainable by the method of the present invention.

Formulations of recombinant Apolipoprotein obtained or obtainable by the present invention or protein bodies comprising Apolipoprotein and having the desired degree of purity may be prepared for storage by mixing with optional pharmaceutically acceptable carriers, excipients or stabilizers (see Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as olyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes; or non-ionic surfactants such as polyethylene glycol (PEG).

Recombinant Apolipoprotein can also be pegylated or bound to polyethylene glycol using known methods. The pegylated Apolipoprotein may be more stable *in vivo* and have a resulting longer half-life in the body when administered to a mammal in need of treatment. Generally, the pharmaceutical compositions may be formulated and administered using methods similar to those used for other pharmaceutically important polypeptides. The recombinant Apolipoprotein may be stored in lyophilized form, reconstituted with sterile water just prior to administration and administered intravenously. Preferably, the pharmaceutical formulation will be administered in dosages that are determined by routine dose titration experiments for the particular condition to be treated.

The following examples are provided as an illustration and not as a limitation. Unless otherwise indicated, the present invention employs conventional techniques and methods of molecular biology, plant biology and plant breeding.

### EXAMPLES

### Example 1 - Materials & Methods

### Cloning and infiltration

Nucleic acid constructs comprising nucleotide sequences encoding gamma-zein wild type gene, fragments and variants thereof are each ligated to a synthetic sequence encoding Apolipoprotein A1 Milano. Where a fragment or variant of gamma-zein is used, the nucleic acid construct further comprise a nucleotide sequence encoding the native gamma-zein signal peptide at the 5' end if it is present in the fragment or variant. For certain experiments, a synthetic nucleic acid sequence encoding a linker comprising a protease cleavage site is also included in the construct, positioned between the gamma-zein and Apolipoprotein A1 Milano coding sequences. The coding sequence of Apolipoprotein A1 Milano has been optimized for expression in plants. The nucleic acid constructs are cloned into a vector at a site where a min35S promoter drives expression of the nucleic acid construct in tobacco plant cells.

Vectors comprising the cloned nucleic acid constructs are introduced into *Agrobacterium tumafaciens* strain Agl1. Agrobacterium cells are grown at 28°C and 250 rpm on a rotary shaker up to an OD600 greater than 1.6. After growth, the bacteria is collected by centrifugation at 8'000 g and 4°C for 15 min and resuspended in infiltration solution containing 10 mM MgCl2 and 5 mM (2-(n-morpholino)-ethanesulfonic acid, MES), final pH 5.6, and OD600= 2.

Plants (*Nicotiana benthamiana*) are grown under normal conditions and individual leaves are infiltrated by standard techniques using a syringe. The leaf is carefully inverted, exposing the abaxial side, and a 1-mL needleless syringe containing the bacterial suspension is used to pressure-infiltrate the leaf intracellular spaces. Six to ten days after infiltration, leaf disks are collected in a heat-sealable pouch, sealed and placed between layers of dry-ice for at least 10 minutes.

### Recovery of protein bodies

Tobacco leaves are ground in liquid nitrogen and homogenized using extraction buffer (50 mM Tris-HCl pH 8,200 mM dithiothreitol (DTT) and optional protease inhibitors (aprotinin, pepstatin, leupeptinc, phenylmethylsulphonyl fluoride and E64 [(N-(N-(L-3-trans-carboxyoxirane-2-carbonyl)-Lleucyl)-agmantine] per gram of fresh leaf material. The homogenates are stirred for 20 min at 4°C and then centrifuged (24000 rpm 20 min, 4°C). The material is filtered through Miracloth by gravity and then centrifuged (200 x g 10 min, 4°C), followed by further centrifugation (6000 x g 10 min, 4°C). The pelleted fraction is washed in 1% Triton X-100 and agitated for 20 minutes, followed by a further centrifugation step (1500 x g 10 min, 4°C).

### Western blot analysis

Proteins are separated on 15% SDS polyacrylamide gel and transferred to nitrocellulose membranes (0.22 ptM) using a semidry apparatus. Membranes are incubated with gamma-zein specific antibody (Ludevid et al. (1985) Plant Sci. 41: 41-48.) and incubated with horseradish peroxidase conjugated antibodies. Immunoreactive bands are detected by enhanced chemiluminescence (ECL western blotting system, Amersham).

### ELISA assays

ELISA assays are conducted for Apolipoprotein A1 Milano quantification on soluble leaf protein extracts and partially purified fusion proteins. Microtiter plates (MaxiSorp, Nalgene Nunc International) are loaded with soluble proteins (100 µl) diluted in phosphate- buffered saline pH 7.5 (PBS) and incubated overnight at 4°C. After washing the wells three times, specific binding sites are blocked with 3% bovine serum albumin (BSA) in PBS-T (PBS comprising 0.1% Tween 20), one hour at room temperature. The plates are incubated with Apolipoprotein A1 Milano antiserum for two hours and after four washes with PBS-T, incubated with peroxidase-conjugated secondary antibodies for two hours. Primary and secondary antibodies are diluted in PBS-T comprising 1% BSA. After washing extensively with PBS-T, the enzymatic reaction is carried out at 37°C with substrate buffer comprising hydrogen peroxide. The reaction is stopped after 10 min with 2N sulphuric acid and the optical density is measured at 450 nm using a Multiskan EX spectrophotometer (Labsystems). The antigen concentration in plant extracts is extrapolated from a standard curve obtained by using Apolipoprotein A1 Milano antiserum.

### Solubilisation of fusion protein accumulated inside protein bodies

The fusion protein is incubated in the buffer chosen for solubilisation of the fusion protein overnight at room temperature.

### Cleavage of fusion protein

A variety of different cleavage agents are analysed in order to cleave Apolipoprotein and without leaving residual amino acids at the N-terminus of Apolipoprotein. Cleavage is carried out for 3 hours at 30 °C in 50mM Tris pH 8.0. The cleavage agents under test are enterokinase, Factor Xa, TEV protease and intein.

### Purification of released Apolipoprotein A1 Milano

Apolipoprotein A1 Milano is resuspended in 20 mM Tris-HCl pH 8.6 and desalted on a PD 10 column (Sephadex G-25 M, Amersham Pharmacia). Desalted protein extracts are subjected to reverse phase immobilized metal ion affinity chromatography to bind gamma-zein using nickel. Apolipoprotein A1 Milano is eluted from the column. The loading buiffer used to load the fusion protein onto the column comprises 50mM Tris pH 8.0, 0.5 mM EDTA and 300 mM NaCl. The eluted Apolipoprotein A1 Milano is then subjected to reverse phase reversed phase fast protein liquid chromatography using an AKTA Explorer reverse phase RESOURCE 1ml reversed phase fast protein liquid chromatography. A first buffer comprising 2% acetonitrile, 0.1% Trifluoroacetic acid and 20 mM beta-mercaptoethanol and a second buffer comprising 80% acetonitrile, 0.1% Trifluoroacetic acid and 20 mM beta-mercaptoethanol is used. The flow rate is adjusted to 1ml/min and a gradient of 0-60% of buffer (80% acetonitrile, 0.1% Trifluoroacetic acid and 20 mM beta-mercaptoethanol) in 10CV and 60-100% of buffer (80% acetonitrile, 0.1% TCA and 20 mM beta-mercaptoethanol) in 20m CV is used. Fractions are eluted in 1ml plus 0.5 ml volumes. The presence of Apolipoprotein A1 Milano in eluted fractions is assessed by 15% SDS polyacrylamide gel electrophoresis and immunoblot detection using Apolipoprotein A1 Milano antiserum. Positive fractions are desalted and concentrated with 5 K NMWL centrifugal filters (BIOMAX, Millipore).

### Example 2 - Expression levels of Apolipoprotein A1 Milano-gamma-zein fusion protein

A gamma-zein-enterokinase-apolipoprotein A1 Milano fusion protein construct (gamma-zein-ApoA1) is prepared as described above and transformed into tobacco plants using Agrobacterium agroinfiltration. Total protein is extracted and quantified by Western blot using gamma-zein-specific antibody. A control experiment using Apolipoprotein A1 Milano expressed under the same conditions without gamma-zein is also carried out (ApoA1). Expression levels from the average of three agroinfiltration events is as follows:
For gamma-zein- Apolipoprotein A1 Milano, the expression levels are between about 3 and 6 g gamma-zein-Apolipoprotein A1 Milano /kg fresh weight.
For Apolipoprotein A1 Milano without gamma zein, the expression levels are between about 2 and 4 g Apolipoprotein/kg fresh weight.
Based on these average results, it was concluded that the expression of gamma-zein-Apolipoprotein A1 Milano is up to about 50% higher than the expression level of Apolipoprotein A1 Milano without gamma-zein.

### Example 3 - Analysis of different non-naturally occurring repeat sequence motifs in gamma zein.

Gamma-zein-Apolipoprotein A1 Milano fusion constructs are prepared using different non-naturally occurring repeat sequence motifs s. The following constructs are used: Gamma-zein peptide only (Gamma-zein-wt); Gamma-zein-(PPPVAL)n; Gamma-zein-(PPPVEL)n; Gamma-zein-(PPPAPA)n; and Gamma-zein-(PPPEPE)n.

The constructs are separately transformed into different tobacco plants using Agrobacterium agroinfiltration. Total protein is extracted and quantified by Western blot using gamma-zein-specific antibody. Expression levels from the average of four agroinfiltration events are as follows:

| Construct tested | Expression level |
|---|---|
| Gamma-zein-wt | 1.0 |
| Gamma-zein-(PPPVAL)n | 1.5 |
| Gamma-zein-(PPPVEL)n | 0.85 |
| Gamma-zein-(PPPAPA)n | 1.81 |
| Gamma-zein-(PPPEPE)n | 1.27 |

Results are represented as relative quantification, relative to gamma-zein without Apolipoprotein A1 Milano.

Three out of the four non-naturally occurring repeat sequence motifs tested (Gamma-zein-(PPPVAL)n, Gamma-zein-(PPPAPA)n and Gamma-zein-(PPPEPE)n significantly increase expression levels of Apolipoprotein A1 Milano as compared to Gamma-zein-wt alone.

Soluble extracts of gamma-zein-(PPPAPA)n and gamma-zein-(PPPEPE)n fusion proteins are obtained from leaves of transgenic tobacco plants and are centrifuged at low speed (about 1500 x g). The precipitated proteins are resuspended in buffer and solubilised. The yields after solubilisation across two trials are as follows:

| Construct tested | Yield after solubilisation compared to Gamma-zein-wt |
|---|---|
| Gamma-zein-wt | 1.0 |
| Gamma-zein-(PPPAPA)n | 7.0 |
| Gamma-zein-(PPPEPE)n | 3.0 |

The use of gamma-zein-(PPPAPA)n and gamma-zein-(PPPEPE)n leads to significantly increased solubilisation yields of Apolipoprotein A1 Milano as compared to the use of gamma-zein-wt with gamma-zein-(PPPAPA)n increasing solubilisation yields of Apolipoprotein A1 Milano by almost 7 fold.

All of the gamma zein peptides tested allow for the accumulation of the fusion protein in dense structures as seen by the recovery of the protein the pellet after low speed centrifugation with no apparent loss of protein in the supernatant.

### Example 5 - Recovery of protein bodies comprising fusion protein

The following method is used to recover the Apolipoprotein A1 Milano fusion protein bodies.

Tobacco leaves are ground in liquid nitrogen and homogenized using extraction buffer (50 mM Tris-HCl pH 8,200 mM dithiothreitol (DTT) and optional protease inhibitors (aprotinin, pepstatin, leupeptinc, phenylmethylsulphonyl fluoride and E64 [(N-(N-(L-3-trans-carboxyoxirane-2-carbonyl)-Lleucyl)-agmantine] per gram of fresh leaf material. The homogenates are stirred for 20 min, centrifuged for 20 minutes (24000 rpm at 4 °C) and agitated for 20 minutes. The mixture is filtered by gravity through one layer of Miracloth. A further centrifugation step (200 x g for 10 minutes at 4 °C) to remove solids and cell debris followed by a second centrifugation step (6000 x g for 10 minutes at 4 °C) to recover fusion protein in the pellet is performed. This is followed by a 1 x wash with 1% Triton X-100 and agitation for 20 minutes followed by a further centrifugation step (1500 x g for 10 minutes at 4 °C). This method also allows for the concentration and enrichment of the protein bodies.

### Example 6 - Solubilisation of fusion protein accumulated inside protein bodies

Different sets of buffers are tested to solubilise the fusion protein. Variables tested include pH, salt, detergent, urea, reducing agent, ratio, time and temperature. A set of conditions identified in this experiment to solubilise the fusion protein was a buffer comprising 50mM Tris pH8, 20mM beta-mercaptoetanol, 1% Triton X-100 and 500 mM NaCl at a ratio of protein bodies:buffer of 1:2 (w/v). The protein bodies are incubated with the buffer overnight at room temperature. Solubilisation yield under these conditions is about 95 %.

### Example 7 - Cleavage of fusion protein

A variety of different cleavage agents are analysed to cleave Apolipoprotein A1 Milano from the fusion protein and without leaving residual amino acids at the N-terminus of Apolipoprotein A1 Milano. Cleavage is carried out overnight at room temperature in 50mM Tris pH 8.0, 0.5mM EDTA, 10mM DTT. When non-proteolytic cleavage agents are used, the buffer may also include 1 x cocktail of protease inhbitiors, EDTA free (Roche). The cleavage agents tested include enterokinase, Factor Xa, TEV protease and intein in combination with various fusion proteins.

Gamma-zein-Glu comprising a (Gly)5 linker is cleaved with TEV protease and results in good cleavage.

Gamma-zein-PAPA comprising a (Gly)5 linker is cleaved with TEV protease and results in good cleavage.

Gamma-zein comprising a (Gly4Ser)3 linker is cleaved with intein and results in good cleavage.

TEV protease results in cleavage of Apolipoprotein A1 Milano from the fusion protein when used with a nucleic acid construct comprising a (Gly)x5 linker and gamma-zein(PPPVEL)n or gamma-zein-(PPPAPA)n. Further analysis has shown that the presence of a linker is not essential for successful cleavage using TEV protease.

The use of an intein also results in cleavage of Apolipoprotein A1 Milano from the fusion protein when used with a nucleic acid construct comprising a (Gly4Ser)3 linker and gamma-zein. Further analysis has shown that the presence of a linker is not essential for successful cleavage using an intein.

### Example 8 -Purification of cleaved Apolipoprotein A1 Milano protein

Various chromatographic methods are tested to identify a method that results in the desired level of purity for Apolipoprotein A1 Milano. A first step comprised the use of Immobilized-metal affinity chromatography followed by a second step of reversed phase fast protein liquid chromatography under the following conditions:

| **System/Technique** | **AKTA Explorer/Reverse Phase** |
|---|---|
| AKTA | 1 |
| Buffer A | 2% acetonitrile, 0.1% TFA and 20 mM β-mercaptoethanol |
| Buffer B | 80% acetonitrile, 0.1% ⁻FA and 20mM β-mercaptoethanol |
| Column | RESOURCE RPC - 1 ml |
| Injection | Loop 5 mL |
| Flow Rate | 1 ml/min |
| Wash | 5 CV (5mL) |
| Gradient | 0 - 60 % 8 in 10 CV - 60 - 100 % B in 20 CV |
| Elution fraction | 1ml - 0.5 mL (microplate) |

| | |
|---|---|
| CV = column volumes. | |

Any publication cited or described herein provides relevant information disclosed prior to the filing date of the present application. Statements herein are not to be construed as an admission that the inventors are not entitled to antedate such disclosures. All publications mentioned in the above specification are herein incorporated by reference. Various modifications and variations of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in cellular, molecular and plant biology or related fields are intended to be within the scope of the following claims.

SEQUENCES

SEQ ID NO. 1

DNA sequence of plant optimised Homo sapiens Apolipoprotein AI-Milano mature peptide

gatgaaccaccacaatctccttgggatagggttaaggatcttgctactgtgtacgtggatgtgcttaaggattctgg
aagggattacgtttctcagtttgagggatctgctcttggaaagcagcttaaccttaagcttctcgataactgggatt
ctgtgacttctactttctctaagctcagagagcaacttggaccagttactcaagagttctgggataacctcgaaaaa
gagactgaaggactcagacaagagatgtctaaggatctcgaagaggttaaggctaaggttcagccatacctcgatga
tttccagaagaagtggcaagaagagatggaactctaccgtcaaaaggttgaaccacttagggctgaacttcaagaag
gtgctaggcaaaagcttcatgagcttcaagagaagctttctccacttggagaagaaatgagagatagggctagggct
catgttgatgctcttaggactcatcttgctccatactctgatgaacttaggcaatgtcttgctgctagacttgaggc
tcttaaagaaaatggtggagctaggcttgctgaatatcacgctaaggctactgagcatctttctacactttccgaga
aggctaaaccagctcttgaagatcttaggcagggacttcttccagttcttgagtctttcaaggtgtccttcttgtct
gctcttgaagagtacactaagaagctcaacactcagtaa

SEQ ID NO. 2

Amino acid sequence of plant optimised Homo sapiens Apolipoprotein AI-Milano mature peptide

DEPPQSPWDRVKDLATVYVDVLKDSGRDYVSQFEGSALGKQLNLKLLDNWDSVTSTFSKLREQLGPVTQEFWDNLEK
ETEGLRQEMSKDLEEVKAKVQPYLDDFQKKWQEEMELYRQKVEPLRAELQEGARQKLHELQEKLSPLGEEMRDRARA
HVDALRTHLAPYSDELRQCLAARLEALKENGGARLAEYHAKATEHLSTLSEKAKPALEDLRQGLLPVLESFKVSFLS
ALEEYTKKLNTQ*

SEQ ID NO. 3

DNA sequence of Zea mays gamma zein (Genbank Accession No. NM_001111884)

1 gcaccagttt caacgatcgt cccgcgtcaa tattattaaa aaactcttac atttctttat
61 aatcaacccg cactcttata atctcttctc tactactata ataagagagt ttatgtacaa
121 aataaggtga aattatgtat aagtgttctg gatattggtt gttggctcca tattcacaca
181 acctaatcaa tagaaaacat atgttttatt aaaacaaaat ttatcatata tcatatatat
241 atatatacat atatatatat atatataaac cgtagcaatg cacgggcata taactagtgc
301 aacttaatac atgtgtgtat taagatgaat aagagggtat ccaaataaaa aacttgttcg
361 cttacgtctg gatcgaaagg ggttggaaac gattaaatct cttcctagtc aaaattgaat
421 agaaggagat ttaatctctc ccaatcccct tcgatcatcc aggtgcaacc gtataagtcc
481 taaagtggtg aggaacacga aacaaccatg cattggcatg taaagctcca agaatttgtt
541 gtatccttaa caactcacag aacatcaacc aaaattgcac gtcaagggta ttgggtaaga
601 aacaatcaaa caaatcctct ctgtgtgcaa agaaacacgg tgagtcatgc cgagatcata
661 ctcatctgat atacatgctt acagctcaca agacattaca aacaactcat attgcattac
721 aaagatcgtt tcatgaaaaa taaaataggc cggacaggac aaaaatcctt gacgtgtaaa
781 gtaaatttac aacaaaaaaa aagccatatg tcaagctaaa tctaattcgt tttacgtaga
841 tcaacaacct gtagaaggca acaaaactga gccacgcaga agtacagaat gattccagat
901 gaaccatcga cgtgctacgt aaagagagtg acgagtcata tacatttggc aagaaaccat
961 gaagctgcct acagccgtct cggtggcata agaacacaag aaattgtgtt aattaatcaa
1021 agctataaat aacgctcgca tgcctgtgca cttctccatc accaccactg ggtcttcaga
1081 ccattagctt tatctactcc agagcgcaga agaacccgat cgacaccatg agggtgttgc
1141 tcgttgccct cgctctcctg gctctcgctg cgagcgccac ctccacgcat acaagcggcg
1201 gctgcggctg ccagccaccg ccgccggttc atctaccgcc gccggtgcat ctgccacctc
1261 cggttcacct gccacctccg gtgcatctcc caccgccggt ccacctgccg ccgccggtcc
1321 acctgccacc gccggtccat gtgccgccgc cggttcatct gccgccgcca ccatgccact
1381 accctactca accgccccgg cctcagcctc atccccagcc acacccatgc ccgtgccaac
1441 agccgcatcc aagcccgtgc cagctgcagg gaacctgcgg cgttggcagc accccgatcc
1501 tgggccagtg cgtcgagttc ctgaggcatc agtgcagccc gacggcgacg ccctactgct
1561 cgcctcagtg ccagtcgttg cggcagcagt gttgccagca gctcaggcag gtggagccgc
1621 agcaccggta ccaggcgatc ttcggcttgg tcctccagtc catcctgcag cagcagccgc
1681 aaagcggcca ggtcgcgggg ctgttggcgg cgcagatagc gcagcaactg acggcgatgt

1741 gcggcctgca gcagccgact ccatgcccct acgctgctgc cggcggtgtc ccccactgaa
1801 gaaactatgt gctgtagtat agccgctggc tagctagcta gttgagtcat ttagcggcga
1861 tgattgagta ataatgtgtc acgcatcac

SEQ ID NO. 4

Translated amino acid sequence of SEQ ID No. 3.

MRVLLVALALLALAASATSTHTSGGCGCQPPPPVHLPPPVHLPPPVHLPPPVHLPPPVHLPPPVHLPPPVHVPPPVH
LPPPPCHYPTQPPRPQPHPQPHPCPCQQPHPSPCQLQGTCGVGSTPILGQCVEFLRHQCSPTATPYCSPQCQSLRQQ
CCQQLRQVEPQHRYQAIFGLVLQSILQQQPQSGQVAGLLAAQIAQQLTAMCGLQQPTPCPYAAAGGVPH

SEQ ID NO. 5

Amino acid sequence of a fragment of gamma-zein

MRVLLVALALLALAASATSTHTSGGCGCQPPPPVHLPPPVHLPPPVHLPPPVHLPPPVHLPPPVHLPPPVHVPPPVH
LPPPPCHYPTQPPRPQPHPQPHPCPCQQPHPSPCQ

SEQ ID No. 6

Amino acid sequence of (PPPAPA)n

APAPPPAPAPPPAPAPPPAPAPPPAPAPPPAPAPPPAPAPPPA

SEQ ID No. 7

Amino acid sequence of (PPPEPE)n

EPAPPPEPEPPPEPEPPPEPEPPPEPEPPPEPEPPPEPEPPPE

SEQ ID No. 8

Amino acid sequence of (PPPVEL)n

VELPPPVELPPPVELPPPVELPPPVELPPPVELPPPVEVPPPVE

SEQ ID No. 9

Amino acid sequence of (PPPVAL)n

VALPPPVALPPPVALPPPVALPPPVALPPPVALPPPVAVPPPVA

SEQ ID No. 10

Amino acid sequence of (PPPVTL)n

VTLPPPVTLPPPVTLPPPVTLPPPVTLPPPVTLPPPVTVPPPVT

SEQ ID No. 11

Amino acid sequence of (PPPAPA)n

PPPAPAPPPAPAPPPAPCPCPAPAPPPCP

SEQ ID No. 12

Amino acid sequence of (PPPEPE)n

PPPEPEPPPEPEPPPEPCPCPEPEPPPCP

## Claims

1. A method for producing Apolipoprotein in a plant comprising incubating or growing a plant into which has been introduced or infiltrated a nucleic acid construct comprising, consisting or consisting essentially of a nucleic acid sequence encoding an Apolipoprotein fusion protein that comprises a fusion protein partner that induces the formation of a protein body in a plant, preferably, wherein the step of introducing or infiltrating the plant is performed prior to the incubating or growing step.

2. The method according to claim 1, wherein the nucleic acid construct comprises:
a first nucleic acid sequence encoding a fusion protein partner that induces the formation of a protein body in a plant optionally, further comprising a nucleic acid sequence encoding one or more non-naturally occurring repeat sequence motifs;
optionally a second nucleic acid sequence encoding an amino acid linker in which a peptide bond therein can be specifically cleaved; and
a third nucleic acid sequence encoding Apolipoprotein, and
wherein said first, second and third nucleic acid sequences are operably linked to each other.

3. The method according to claim 1 or claim 2, wherein the nucleic acid sequence further comprises a nucleic acid sequence encoding a fusion protein partner that directs the protein towards the endoplasmic reticulum of a plant cell, preferably a signal peptide.

4. The method according to any of the preceding claims, comprising the additional step of:
recovering the protein body comprising the Apolipoprotein fusion protein from the plant, preferably wherein said step comprises the steps of:
(i) homogenising the plant material;
(ii) centrifuging the homogenised plant material at low speed, preferably, about 200 *x g*;
(iii) centrifuging the homogenised plant material at a higher speed than step (ii), preferably, about 6000 x g; and
(iv) recovering the protein bodies comprising the fusion protein in the pelleted fraction.

5. The method according to any of the preceding claims, comprising the further step of:
solubilising the Apolipoprotein fusion protein, preferably, wherein said solubilisation step comprises the use of a mixture comprising, consisting or consisting essentially of a reducing agent , preferably, beta-mercaptoethanol, a non-ionic surfactant, preferably Triton X-100 and optionally salt, preferably, sodium chloride.

6. The method according to any of the preceding claims, comprising the further step of:
releasing Apolipoprotein from said fusion protein partner, preferably, wherein a protease, preferably, TEV protease, or a protein splicing means, preferably an intein, is used to release Apolipoprotein from said fusion protein partner.

7. The method according to any of the preceding claims, comprising the further step of:
purifying the released Apolipoprotein.

8. The method according to claim 7, wherein said step comprises:
(i) contacting the Apolipoprotein fusion protein with an immobilized metal ion affinity chromatography column to immobilise the prolamin protein;
(ii) eluting the Apolipoprotein; and
(iii) further purifying the eluted Apolipoprotein using reversed phase chromatography, preferably reversed phase reversed phase fast protein liquid chromatography.

9. A nucleic acid construct comprising, consisting or consisting essentially of:
a first nucleic acid sequence encoding a prolamin protein that induces the formation of a protein body in a plant, preferably, gamma-zein;
optionally a second nucleic acid sequence encoding a spacer, preferably, a cleavage recognition site; and
a third nucleic acid sequence encoding Apolipoprotein,
wherein said first, second and third nucleic acid sequences are operably linked to each other.

10. The nucleic acid construct according to claim 9, further comprising:
a regulatory nucleotide sequence that regulates the transcription of said nucleic acid sequences.

11. A vector comprising the nucleic acid construct according to claim 9 or claim 10.

12. A fusion protein comprising, consisting or consisting essentially of:
(i) an amino acid sequence encoding a prolamin protein that induces the formation of a protein body in a plant, preferably, gamma-zein optionally, wherein said amino acid sequence further comprises an amino acid sequence encoding one or more non-naturally occurring repeat sequence motifs;
(ii) optionally an amino acid sequence encoding a linker in which a peptide bond therein can be specifically cleaved; and
(iii) an amino acid sequence encoding Apolipoprotein.

13. A plant or plant material derived therefrom comprising the nucleic acid sequence according to claim 9, or the nucleic acid construct according to claim 10, or the vector according to claim 11, or the fusion protein according to claim 12.

14. A plant protein body comprising the fusion protein according to claim 12.

15. The method according to any of claims 1 to 8, the nucleic acid sequence according to claim 9, the nucleic acid construct according to claim 10, the vector according to claim 11, the fusion protein according to claim 12, the plant according to claim 13, or the plant protein body according to claim 14 wherein the Apolipoprotein is a mutant thereof, preferably, Apolipoprotein A1 Milano.
